# EUROPEAN PATENT APPLICATION

(11) **EP 1 386 968 A1**
(43) Date of publication of application: **04.02.2004**
(21) Application number: 02713310.7
(22) Date of filing: 09.04.2002
(51) Int. Cl.: C12N 15/85, C12N 15/10, G01N 33/50, G01N 33/68, A01K 67/027

(54) **GENE EXPRESSION CONTROLLING UNIT AND UTILIZATION THEREOF**

(30) Priority: 09.04.2001 JP 2001109445
(71) Applicant: Gencom Corporation, Machida-shi, Tokyo 194-8511 (JP)
(72) Inventor: YAMAMOTO, Mutsuya, Machida-shi, Tokyo 194-8511 (JP); WATANABE, Dai, 605, Rowaiyaru Muromachi, Nakagyo-ku, Kyoto-shi, Kyoto 604-0000 (JP); TERANISHI, Y., Mitsubishi Kagaku Inst. Lifesc., Machida-shi, Tokyo 194-8511 (JP); NAKANISHI, Shigetada, Kyoto-shi, Kyoto 606-0042 (JP)
(74) Representative: TER MEER STEINMEISTER & PARTNER GbR
(86) International application number: PCT/JP2002/003537
(87) International publication number: WO 2002/086131

(57) **Abstract**

An object of the present invention is not to delete certain nerve cell by irreversible cell death, but to provide a very effective tool for analyzing the network of the cerebral nervous system by controlling reversibly the expression of a protein having a synaptic transmission-controlling activity in certain nerve cell. The present invention provides a DNAwhich comprises a DNA encoding a protein having a synaptic transmission-controlling activity and has a construct capable of controlling reversibly the expression of the protein having a synaptic transmission-controlling activity in certain nerve cell.

## Description

### Technical Field

The present invention relates to a method for analyzing a function of certain nerve cell. More specifically, the present invention relates to a DNA having a construct which can reversibly control the expression of a protein having a synaptic transmission-controlling activity in certain nerve cell, a host wherein this DNA is introduced, and a method for analyzing the function of certain nerve cell by reversibly controlling the expression of a protein having the synaptic transmission-controlling activity in certain nerve cell in the host wherein this DNA is introduced.

### Background of Invention

Conventionally, for the purpose of revealing the brain function and preparing a model animal of a disease of a nervous system, many studies have been carried out by applying physiological and pharmacological approaches. These examples include administration of reserpine, which is an adrenergic neuronal blocking agent, forpreparingadepressionmodelanimal, and local administration of a substance named MPTP (1-methyl-4-phenyl-1,2,5,6-tetrahydropyridine) to substania nigra to injure dopamine-producing cells, for preparing a model animal of Parkinson's disease. However, a brain is an environment containing various types of cells such as a cell having a property of [A), a cell having a property of [B] and a cell having a property of [C]. Therefore, even if a drug is administered or a physical operation is conducted, with the purpose of operating only the [A] cell and leaving the other cells such as the [B] cell and [C] cell as they are, it is actually inevitable that not only the [A] cell but also the other cells are considerably influenced. As described above, a conventional experiment of administering an agonist or antagonist of a functional protein and a conventional site-breakdown experiment always have a fatal defect, that is, an influence of the experimental operation is nonspecifically given to a wide range of a tissue. Thus, it was impossible to carry out a functional analysis of certain cell in vivo and an analysis of a disease caused by a function disorder of certain cell.

On the other hand, by means of molecular biological approaches which have been remarkably developed in recent years, a transgenic mouse and a knock-out mouse can be created, and the expression amount of a gene in an individual can be artificially changed. However, this approach is restricted to the analysis of a phenotype when the expression of a gene increases or when the expression of a gene disappears, that is, the "molecular" function such as how the gene works in vivo. In other words, there is no effective means for specifically analyzing the function of a nerve cell, which is located in a higher level than amolecule and is a basic unit of a nerve network.

Under such the situation, Watanabe, Nakanisi, et al. who is the inventors of the present invention, have developed IMCT (Immunotoxin-Mediated Cell Targeting) method (Watanabe, D., et al. (1998) Cell 95: 17-27). IMCT method is an epoch-making method wherein a transgenic mouse which expresses a fusion protein of human IL-2 receptor and a marker in certain region of a brain is created by a molecular biological approach, and an antibody toxin which specifically recognizes human IL-2 receptor is administered to this mouse, thereby achieving the breakdown and lack of certain cell in any growth stage.

However, IMCT method has a limit, and is not always sufficient for the analysis of the function of nerve cells. A cerebral nervous system is a highly plastic organ. If a nerve cell is lacked, compensation and adaptation by other cells occur during a long-term observation. In the IMCT method, the nerve cell is reversibly lacked by cell death. Therefore, the mouse can be converted from a normal state to a lacking state, but the mouse cannot be recovered from the lacking state to the normal state. Hence, an irreversible method of nerve cell elimination such as IMCT method has a problem that a cell function hindered by compensation and adaptation phenomena cannot be exactly evaluated.

For analyzing the cerebral nervous system network more accurately and studying on pathology, diagnosis and therapy for cerebral nerve diseases, it is indispensable to develop a method for reversibly controlling the function of a nerve cell.

A toxic protein which is produced by Clostridium tetani and Clostridium botulinum, when taken into a nerve cell, works as a protease to digest specifically a protein (SNARE: Soluble N-ethylmaleimide-sensitive fusion protein Attachment proteins Receptor) having a function of releasing a neurotransmitter. As a result, the nerve cell which was influencedby this neurotoxic protein cannot release a neurotransmitter, and the transmission of information to postsynaptic cells is inhibited without cell death. Therefore, this toxic protein derived from Clostridium tetani and Clostridium botulinum is expected to be useful as a protein having a synaptic transmission-controlling activity. However, when Clostridium tetani toxin is tried to be constitutively expressed in a mouse individual, abnormality occurs in sperm formation (Eisel, U. et al. (1993) EMBO J. 12: 3365-3372), and thus a transgenic mouse in which Clostridium tetani toxin is expressed in a nervous system cannot be created. Consequently, a simple application is considered to be difficult.

In a stream of development of molecular biology, a method is being developed for operating and controlling reversibly the gene expression by placing the cell in certain condition. For example, there are methods using a tetracycline expression control system (Gossen, M. and Bujard, H. (1992) Proc. Natl. Acad. Sci. USA 89: 5547-5551; US Patent No. 5,464,758) , a heavy metal (Mayo, K. E. et al. (1982) Cell 29: 99-108; Brinster, R. L. et al. (1982) Nature 296: 39-42; Searle, P. F. et al. (1985) Mol. Cell Biol. 5: 1480-1489) , or heat shock (Nouer et al. (1991) Heat shock response, Nouer, L. , Florida, Boca Raton, CTC Press 167-220) , a promoter which is responsive to a hormone (Lee, F. et al. (1981) Nature 294: 228-232; Hynes N. E., et al. (1981) Proc. Natl. Acad. Sci. USA 78: 2038-2042; Klock, G. et al. (1987) Nature 329: 734-736; Israel, D. I. and Kaufman, R. J. (1989) Nucleic Acid Res. 17: 4589-4604), and a method of using lac operator/repressor system of Escherichia coli (Hu, M. C-T. and Davidson, N. (1987) Cell 48: 555-566). However, a method for controlling reversibly the function of a nerve cell by using these methods for the control of gene expression, has not yet been developed.

### Disclosure of the Invention

According to the present invention, certain nerve cell is not lacked by irreversible cell death. The problem to be solved by the present invention is to provide a very effective tool for analyzing the network of the cerebral nervous system by controlling reversibly the expression of a protein having a synaptic transmission-controlling activity in certain cell.

The present inventors have used a tetracycline dependent transcription control system by using GABA_{A} α 6 promoter (Jones, et al. (1996) J. Neurochem. 67: 907-916; Bahn, S. et al. (1997) Proc. Nat'l Acad. Sci. USA 94: 9417-9421) which is activated specif ically in certain nerve cell, and have created a transgenic mouse by microinjecting a DNA which has a construct capable of reversible control of the expression of the neurotoxin gene derived from Clostridium tetani and Clostridium botulinum, into a murine fertile egg by the aforementioned system. By administering or not administering doxycycline, a tetracycline analog, to this mouse, it was found that the release of a neurotransmitter is controlled in a cell in which GABA_{A} α 6 promoter is specifically activated. The present invention has been achieved on the basis of such findings.

Thus, the present invention provides:
(1) ADNAwhichcomprises a DNA encoding a protein having a synaptic transmission-controlling activity and has a construct capable of controlling reversibly the expression of the protein having a synaptic transmission-controlling activity in certain nerve cell;
(2) A gene expression control unit which comprises (a) a DNA construct which can reversibly control the expression of a protein having a synaptic transmission-controlling activity in certain nerve cell and (b) a DNA which is ligated in such a way that the DNA is placed under the control of said DNA construct and encodes the protein having a synaptic transmission-controlling activity;
(3) a gene expression control unit which comprises (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity;
(4) The gene expression control unit according to the above (3) wherein the protein having a neurotransmitter release - controlling activity is a neurotoxin;
(5) A host having the DNA or the gene expression control unit according to any one of the above (1) to (4);
(6) A method for analyzing the function of certain nerve cell, wherein the expression of the protein which is contained in the host according to the above (5) and has a neurotransmission function-controlling activity in certain nerve cell, is reversibly controlled, and the change of a phenotype appearing in the host is analyzed;
(7) Amethod for analyzing the function of a biological functional molecule, wherein the expression of the protein which is contained in the host according to the above (5) and has a neurotransmission function-controlling activity in certain nerve cell is reversibly controlled, and the physical and chemical changes of the biological functional molecule in the nerve cell or a cell associated therewith are analyzed;
(8) A method for analyzing the function of certain nerve cell, wherein (1) (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity, are introduced into a host, and (2) the change of a phenotype appearing in the host is analyzed by controlling the presence or absence of a specific stimulus against the host or the nerve cell or its strength;
(9) Amethod for analyzing the function of a biological functional molecule, wherein (1) (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity, are introduced into a host, and (2) the physical and chemical changes of the biological functional molecule in certain nerve cell or the cell associated therewith is analyzed by controlling the presence or absence of a specific stimulus against the host or the nerve cell or its strength;
(10) A method according to the above (8) or (9), wherein the protein having a neurotransmitter release-controlling activity is a neurotoxin;
(11) A fertilized egg, an embryonic stem cell and a nerve stem cell of an non-human animal which has (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity;
(12) The fertilized egg, the embryonic stem cell and the nerve stem cell according to the above (11) wherein the non-human animal is a rodent;
(13) The fertilized egg, the embryonic stem cell and the nerve stem cell according to the above (11) or (12), wherein the non-human animal is a mouse;
(14) A transgenic non-human animal which is obtained by development from fertilized egg or the embryonic stem cell according to any one of the above (11) to (13), and an offspring thereof;
(15) A transgenic animal cell which is obtained from the transgenic animal according to the above (14);
(16) A nerve cell which is obtained by differentiation of the nerve stem cell according to any one of the above (11) to (13):

### BRIEF DESCRIPTION OF DRAWINGS

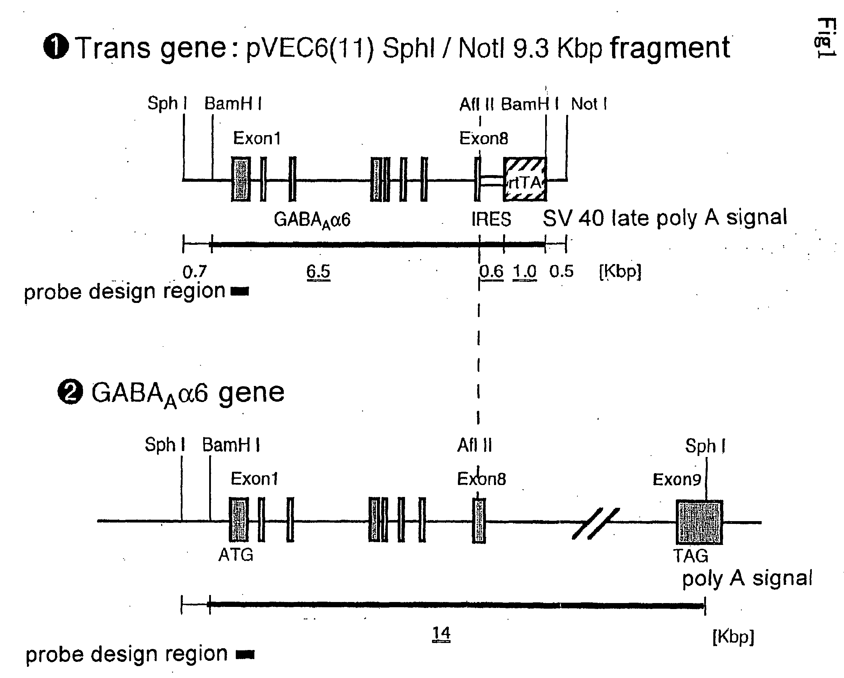

Fig. 1 shows a DNA encoding a tetracycline dependent transcription control factor which was prepared in the Examples of the present invention. ① shows the structure of SphI/ NotI 9.3 kbp fragment of pVEC6(11) vector which was used for the microinjection into a murine fertilized egg. ② shows the structure of endogenous GABA_{A}α6 gene. In both ① and ②, the gray wide frame shows an exon region of GABA_{A} α 6, and the white narrow frame shows IRES, and the diagonally shaded wide frame shows the rtTA gene. Numerals represent sizes (kbp) of DNAs of each region, and the bold line representing the probe-defined region in the lower part shows the region corresponding to the probe sequence used for Southern hybridization.

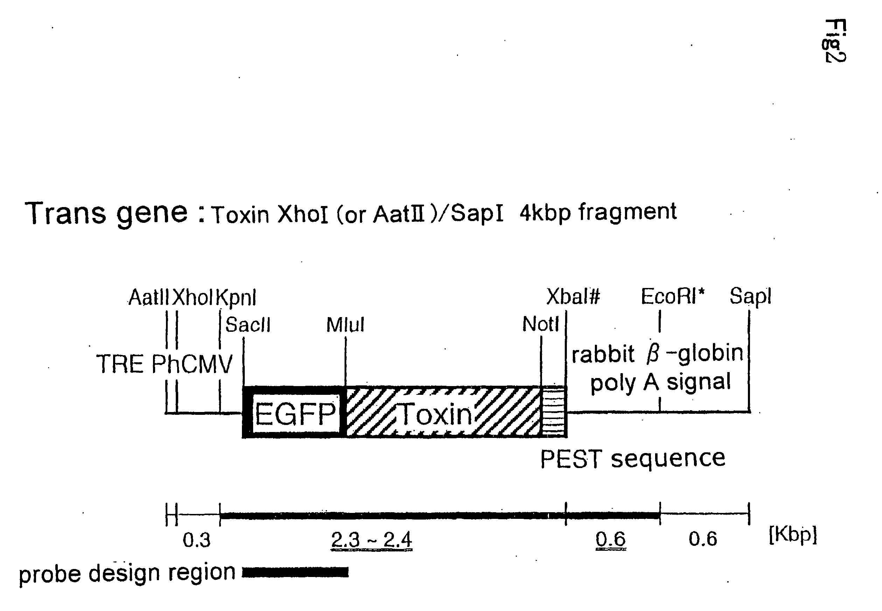

Fig. 2 shows the DNA which was prepared in the Examples of the present invention and encodes a synaptic transmission-controlling protein. Numerals represent sizes (kbp) of DNAs of each region, and the bold line representing the probe-defined region in the lower part shows the region corresponding to the probe sequence used for Southern hybridization.
XhoI: 1 site in BoNT.A, BoNT. B, BoNT.E, and TeNT
BoNT.CI contains another site in a different region.
Xba1#: 1 site in BoNT.A, BoNT.B, and BoNT.E
BoNT.CI and TeNT contain another site in a different region.
EcoRI: 1 site in BoNT.A, BoNT.B, BoNT.CI, and TeNT
BoNT.E contains another site in a different region.

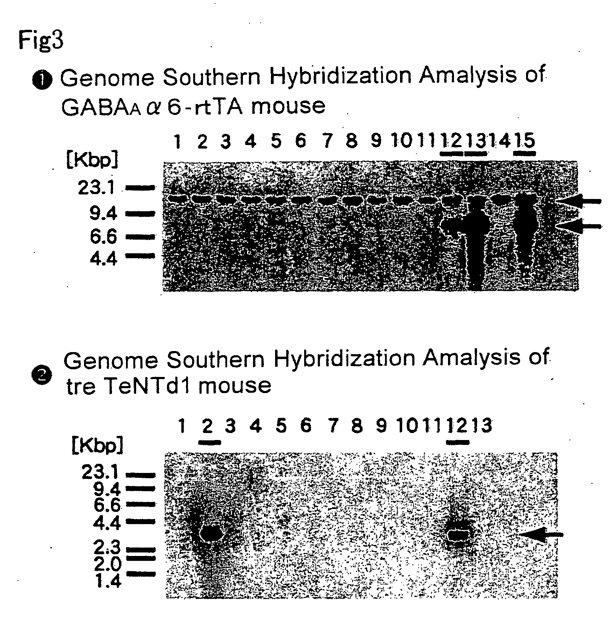

In Fig.3, ① shows the result of genome Southern hybridization analysis of the transgenic mouse into which GABA_{A} α 6-rtTA was introduced. ② shows the result of genome Southern hybridization analysis of the transgenic mouse into which treTeNTd1 was introduced. In both ① and ②, upper numerals represent individual numbers of the transgenic mouse which is a test animal, and underlines attached to the individual numbers show the transgene-positive mouse. A size marker of the DNA is presented on the left side of the figure. A long arrow indicates the position of the signal derived from the endogenous gene, and a short arrow indicates the position of the signal derived from the transgene. A transgene-positive line is shown with the underline attached to a number.

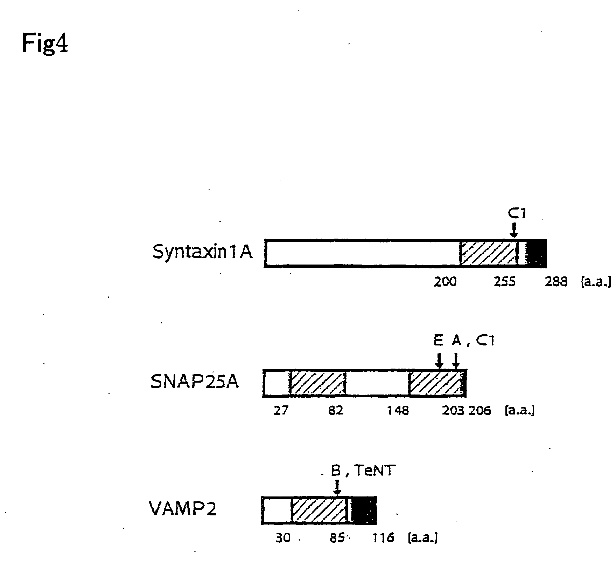

Fig.4 is shows the relation of the structure of the substrate protein of a neurotoxin with the cleavage site by the neurotoxin used in the Examples of the present application. In the figure, each letter of A, B, Cl and E represents serotype of BoNT which is the neurotoxin, and the arrows attached show the sites in which each neurotoxins cleave the substrate. The region diagonally shaded shows Four-helices bundle region (Fasshauer, D., et al. (1998) Proc. Natnl Acad. Sci. USA 95: 15781-15786), and solid region shows a transmembrane region. Numerals of the lower part represent the N-terminal amino acid residue number of the each regional boundary.

Fig. 5 shows the result of immunoblotting for the analysis of substrate protein cleavage activity of the toxin as to the neurotoxin protease and its substrate protein which are coexpressed in a host cell. A molecular weight marker of the protein is presented on the left side of the figure. The long arrow indicates the position of the site of a target protein which was not cleaved, and a short arrow indicates the position of the site of a target protein which was cleaved.

Fig. 6 shows the result of RNA blotting analysis of the GABA_{A} α 6-rtTA gene-introduced mouse.

Fig. 7 shows the result of in situ hybridization analysis in the GABA_{A} α 6-rtTA gene-introduced mouse.

Fig. 8 shows the result of the detection of the expression induction and expression stopping of a neurotoxic protein in GABA_{A}α 6-rtTA gene- and treToxin gene- double transgenic mouse by immunoblotting.

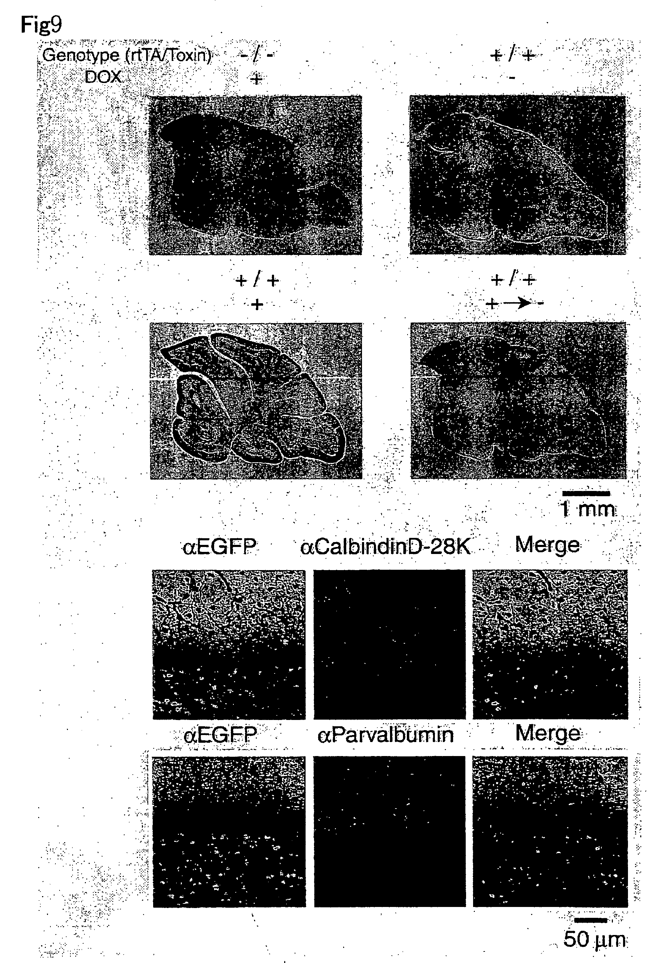

Fig. 9 shows the result of the analysis wherein cerebellar sections of a wild mouse (6-week old) to which doxycycline was administered for 1 week (Fig. 9A: genotype: -/-, DOX: +), the double transgenic mouse (6-week old) to which no doxycycline was administered (Fig. 9B: genotype: +/+, DOX: -), the double transgenic mouse (6-week old) to which doxycycline was administered for 1 week (Fig. 9C: genotype: +/+, DOX: +), the double transgenic mouse (10-week old) to which doxycycline was administered for 2 week followed by eliminating doxycycline for 3 weeks (Fig. 9D: genotype: +/+, DOX: + → -), were used, and the primary antibody reaction was carried out by using an anti-EGFP antibody, and the ABC method was carried out for the secondary reaction and later.

Fig. 10 shows the result of analysis of behavior by Rota-Rod test using the mouse.

Fig. 11 shows the result of analysis of behavior by balance beam test using the mouse.

### The Best Mode for Carrying Out the Invention

### (1) A protein having a synaptic transmission-controlling activity, and a DNA encoding the protein.

In the present invention, the protein which is controlled for its expression and has a synaptic transmission-controlling activity (which may be hereinafter referred to as "synaptic transmission-controlling protein") is a protein having an activity of controlling the signal transduction between certain nerve cell and other cells in vivo. Preferred example thereof is a protein which can directly control the release of a neurotransmitter (which is hereinafter referred to as "neurotransmitter release-controlling protein"). However, any protein can be used so long as the synaptic transmission can be reversibly controlled by regulating the degree of its expression.

As the synaptic transmission-controlling protein such as the neurotransmitter release-controllingprotein, which is used in the present invention, any protein may be used so long as it can reversibly suppress or promote the release of a neurotransmitter when it is introduced into a living host or is eliminated from the living host. For example, the proteins which can be used include a neurotoxin, a factor influencing sensitivity, metabolism or localization of aneurotoxin, a factor influencing a cycle of vesicles containing a neurotransmitter, a factor influencing synthesis, decomposition, modification or localization of a neurotransmitter, a factor influencing an electric potential and an ion concentration in a cell related to the release of a neurotransmitter, and a factor influencing a signal related to the release of a neurotransmitter.

Of these proteins, it is preferable to use a neurotoxin. The neurotoxin includes a toxin protease (TeNT) derived from Clostridium tetani and the toxin protease (BoNT) derived from Clostridium botulinum. These neurotoxins may be those derived from any types or varieties of Clostridium tetani or Clostridium botulinum. The toxin proteases are preferably those derived from Clostridium tetani Harvard A-47 ; deposition number KZ1174, Nippon Saikingaku Zassi (1995) vol. 50, no. 4: 1023) or derived from Clostridium botulinum [BoNT.A (Binz, T., et al., (1990) J. Biol. Chem., 265, 9153-9158) , BoNT.B (Kurazono, H., et al., (1992) J. Biol. Chem., 267, 14721-14729) , BoNT.Cl (Hauser, D., et al. , (1990) Nucleic Acids Res. , 18, 4924) , BoNT.D (Binz, T., et al., (1990) Nucleic Acids Res., 18, 5566), BoNT.E (Poulet, S., et al., (1992) Biochem. Biophys. Res. Commun . , 183, 107-113), BoNT.F (East, A. K. et al., (1992) FEMS Microbiol. Lett., 96, 225-230) , BoNT.G (Campbell, K. , et al. , (1993) Biochem. Biophys. Acta. , 1216, 487-491), TeNT (Eisel, U. , et al. , (1986) EMBO J., (1986) 5, 2495-2502)]. Usable amino acid sequences of these synaptic transmission-controlling protein includes those described in the aforementioned references. So long as the synaptic transmission-controlling activity is maintained, those having substitution, deletion, addition, or inversion of 1 or several amino acids can be used as the synaptic transmission-controlling protein. If necessary, a marker and a tag sequence can be added to the N-terminal or a C-terminal of the protein or the like, and internal insertion is also possible. The marker which can be used includes, for example, GFP (Green Fluorescent Protein), β-galactosidase, and luciferase. The tag sequence may be any one which is ordinarily used as an epitope tag, and the example thereof includes Flag tag, HA tag, and HIS tag. In addition, if necessary, a sequence which influences intracellular transport and localization of the protein and a sequence which regulates stability of the protein, can be added to the N-terminal or a C-terminal of the protein, or can be inserted within the protein. The sequence which influences intracellular transport and localization can be used for, for example, the method described in Callahan, C. A. and Thomas, J. B. (1994) Proc. Natnl Acad. Sci. USA 91: 5972-597.6, in which targeting to an axon was carried out by using the amino acid sequence of tau protein, or the method described in Moriyoshi, K. et al. (1996) Neuron 16: 255-260, in which targeting to a cell membrane was carried out by using Growth-associated protein-43 (GAP-43) or an about 20 amino acid fragment in c-Ha-R as. Usable sequence capable of regulating stability of the protein includes PEST sequence such as mouse ornithine decarboxylase (Ghoda, L. , et al. (1989) Science 243: 1493-1495; Li, X., et al. (1998) J. Biol. Chem. 273: 34970-34975).

The DNA encoding the synaptic transmission-controlling protein, which is used in the present invention, can be obtained, for example, from the cell in which the aforementioned synaptic transmission-controlling protein is expressed. Any types of the DNA may be used so long as it encodes the protein having the synaptic transmission-controlling activity, and either genome DNA or cDNA may be suitable, and cDNA is preferable. The cDNA may contain the coding region only, and may contain the non-coding region, and can be obtained from mRNA in the cell, where the synaptic transmission-controlling protein is expressed, or from cDNA libraries by methods known per se such as RT-PCR (Reverse Transcription polymerization chain reaction) , PCR or hybridization method.

The DNA encoding the synaptic transmission-controlling protein, which is used in the present invention, includes not only DNAs obtained by the manner as described above, but also DNAs preparedby ligatinga sequence for regulatingtranscription and translation, or intracellular localization of mRNA (Mayford, M., et al. , (1996) Proc. Natl. Acad. Sci. USA, 93, 13250-13255; Mori, Y., et al., (2000) Nature Neurosci., 3, 1079-1084; Oleynikov, Y., and Singer, R. H., (1998) Trends Cell Biol., 8, 381-383) or stability of mRNA(Chen, C. -Y. A., and Shyu, A.-B., (1995) Trends Biol. Sci., 20, 465-470) , or for editing the RNA and the DNA in 3' side or 5' side or inside of the DNA, so long as the synaptic transmission-controlling activity of the protein encoded by the DNA is not inhibited. The DNA sequence having a transcription-controlling activity includes poly A signal, a promoter, an enhancer, and a silencer. The DNA having a translation-controlling activity includes the IRES (Jang, S. K., et al., (1989) J. Virol. 63, 1651-1660) and Kozak sequence (Kozak, M., (1986) Cell, 44, 283-292). The DNA sequence for editing the RNA and the DNA includes a splicing site, 1 oxP sequence (Sternberg, N., and Hamilton, D., (1981) J. Mol. Biol., 150, 467-486; Sauer, B., and Henderson, N., (1988) Proc. Natl. Acad. Sci. USA, 85, 5166- 5170; Gu, H. , et al. , (1994) Science, 265, 103-106) , FRT sequence (Golic, K. G. , andLindquist, S. L. , (1989) Cell, 44, 499-509), a transposon, a chromosome homologous sequence. In addition, DNA of another gene may be ligated. Among these sequences, those prepared by ligating poly A signal sequence to the 3' side of a translation region DNA can be preferably used. Usable poly A signal sequence may be one contained in 3' non-translation region of the cDNA which was used. Alternatively, an exogenous poly A signal sequence such as Rabbit β-globin polyA signal sequence may be used.

Preferred examples of such DNA encoding the synaptic transmission-controlling protein include light chain genes of BoNT.A (Binz, T. , etal., (1990) J. Biol. Chem. , 265, 9153-9158) , BoNT.B (Kurazono, H., et al., (1992) J. Biol. Chem., 267, 14721-14729) , BoNT.Cl (Hauser, D. , et al. , (1990) Nucleic Acids Res., 18, 4924) , BoNT.D (Binz, T., et al. , (1990) Nucleic Acids Res., 18, 5566), BoNT.E (Poulet, S., et al., (1992) Biochem. Biophys. Res. Commun., 183, 107-113), BoNT.F (East, A. K. et al., (1992) FEMS Microbiol. Lett., 96, 225-230) , BoNT.G (Campbell, K., et al., (1993) Biochem. Biophys. Acta., 1216, 487-491) , and TeNT (Eisel, U., et al. , (1986) EMBO J. , (1986) 5, 2495-2502; GenBank Accession No.X04436). More preferable examplesinclude those obtained by adding PEST sequence and Rabbit β -globin polyA signal sequence to the 3' terminal of these DNAs.

### (2) The expression control unit of the synaptic transmission-controlling protein.

By adding the expression control region which can reversibly control the expression in certain nerve cell to the DNA encoding the synaptic transmission-controlling protein as described above (which may be hereinafter referred to as "synaptic transmission-controlling DNA"), the expression control unit of the synaptic transmission-controlling protein according to the present invention (which may be hereinafter referred to as "a gene expression control unit" or "an expression control unit of the synaptic transmission-controlling protein") can be prepared.

Examples of such gene expression control unit include those containing (a) a DNA construct which can reversibly control the expression of the protein having the synaptic transmission-controlling activity in certain nerve cell, and (b) a DNA which is ligated in such a way that it is controlled by the DNA construct, and encodes the protein having the synaptic transmission-controlling activity.

Certain nerve cell means the cell in which the synaptic transmission-controlling protein is expressed in the cell to change a network between said cells and other cells associated therewith. Specific examples thereof include granule cells of cerebellum.

The system for reversibly control the expression of the synaptic transmission-controlling protein in these certain nerve cells may be any systems, so long as the expression of a gene can be reversibly controlled by a specific condition and a reversible control can be carried out specifically in certain nerve cell. Among these systems, for the system for reversibly controlling the expression of a gene, any method known per se can be used. Examples thereof include a tetracycline expression control system for controlling the gene expression in accordance with presence or absence of tetracycline (Gossen, M. and Buj ard, H. (1992) Proc. Nat'l Acad. Sci. USA 89: 5547-5551; US Patent No. 5,464,758) , a method using a promoter responsive to a heavy metal (Mayo, K. E. et al. (1982) Cell 29: 99-108; Brinster, R. L. et al. (1982) Nature 296: 39-42; Searle, P. F. et al. (1985) Mol. Cell Biol. 5: 1480-1489), heat shock (Nouer et al. (1991) Heat shock.response, Nouer, L., Florida, Boca Raton, CTC.Press 167-220) or a hormone (Lee, F. et al. (1981) Nature 294: 228-232; HynesN. E. , etal. (1981) Proc. Nat'lAcad. Sci. USA78: 2038-2042; Klock, G. et al. (1987) Nature 329: 734-736; Israel, D. I. and Kaufman, R. J. (1989) Nucleic Acid Res. 17: 4589-4604), and a method using the lac operator/repressor system of Escherichia coli (Hu, M. C-T. and Davidson, N. (1987) Cell 48: 555-566).

For selective activation of these systems for reversibly controlling the expression of a gene in an object nerve cell only, for example, the system may be ligated to be placed under the control of the promoter which is activated only in certain nerve cells, or a specific condition for reversibly controlling the gene expression in the system may be selectively applied in certain nerve cells. Whether or not the expression control unit according to the present invention is selectively activated only in the object nerve cell, can be examined by transducing the unit into a proper host and analyzing the change of an amount of expression of the synaptic transmission-controlling protein or an mRNA encoding the protein by, for example, immunoblotting, immunohistochemical staining, RNA blotting, in situ hybridization or the like under a specific condition for reversibly controlling the expression of a gene.

The method of specific activation of reversible control of the protein expression in certain nerve cell by means of the expression control unit according to the present invention will be described in detail with reference to the following examples.

### (i) Construction (1) of the expression control unit of the synaptic transmission-controlling protein in certain nerve cell using the tetracycline expression control system

The gene expression control unit according to the present invention includes that using the tetracycline expression control system as mentioned above. Examples of the specific constitution of the unit include (a) a DNA obtained by ligating a tetracycline responsive sequence and a promoter controlled by the tetracycline responsive sequence at the 5' upstream side of the synaptic transmission-controlling DNA according to the present invention, and (b) a DNA obtained by ligating a DNA encoding a tetracycline expression-regulating factor in such a way that it is placed under the control by the promoter sequence which is activated specifically to certain nerve cell. These two DNA fragments may be independent DNA fragment respectively, or may be constructed by ligating them, or may be constructed as a plasmid wherein they are inserted.

The ligation of these DNAs can be preferably carried out as described above. However, as long as the expression of the synaptic transmission-controlling protein can be reversibly controlled in the cell where the expression control unit according to the present invention is introduced, by controlling the presence or absence of tetracycline or its amount, the site of ligation may be moved to other site or the sequence may be changed. Moreover, in addition to the above described constitution, it is preferable to insert at least one proper intron.

The tetracycline responsive sequence as described above may be any DNA, so long as it can be ligated with the tetracycline expression control factor and has the function of activating the promoter in the downstream by this ligation. Specifically, for example, a tetracycline operator (Gossen, M. and Bujard, H., (1992) Proc. Natl. Acad. Sci. USA, 89, 5547-5551) is used. The promoter ligated to the downstream of the responsive sequence may be any promoter, so long as it has no activity in itself and can be controlled by the tetracycline responsive sequence. The specific examples include a CMV minimum promoter.

The tetracycline expression-regulating factor includes a factor which induces a gene expression controlled by the tetracycline responsive sequence by administration or no administration of tetracycline or its derivative. Specific examples thereof include rtTA (Reverse tetracycline-controlled transactivator: Gossen, M., et al., (1995) Science, 268, 1766-1769), and tTA (Tetracycline-controlled transactivator: Gossen, M., and Bujard, H., (1992) Proc. Natl. Acad. Sci. USA, 89, 5547-5551).

The DNA (which may be hereinafter referred to as "tetracycline expression-regulating DNA") which encodes the tetracycline expression-regulating factor as described above is ligated in such a way that it is placed under the control of the promoter which is activated in certain nerve cell. Specifically, usable examples include a DNA which is prepared by ligating the promoter sequence which is activated in certain nerve cell to the 5' side upstream of the tetracycline expression-regulating DNA, and a DNA in which the tetracycline expression-regulating DNA is located in the downstream of the endogenous promoter on the chromosome of the cell which is introduced by homologous recombination and is activated in certain nerve cell. To the 5' side upstream or the 3' side downstream of the tetracycline expression-regulating DNA, a sequence for regulating transcription and translation or intracellular localization or stabilization of the mRNA or editing the RNA or the DNA, may be ligated, so long as the activity of the protein encoded by the DNA is not inhibited. Examples of these DNAs include those attached to the synaptic transmission-controlling DNA according to the present invention, which is described in (i).

The promoter which is activated in certain nerve cell includes GABA_{A} α 6 promoter (Jones, A., et al., (1996) J. Neurochem., 67, 907-916; Bahn, S., et al. (1997) Proc. Natl. Acad. Sci. USA, 94, 9417-9421). The 3' side downstream of the DNA sequence of the promoter may contain a gene DNA controlled by the promoter. In this case, the IRES (Internal ribosomal entry site) may be inserted between the gene as described above and the tetracycline expression-regulating DNA according to the present invention.

### (ii) Construction (2) of the expression control unit of the synaptic transmission-controlling protein in certain nerve cell using the tetracycline expression control system

A further example of the tetracycline expression control unit according to the present invention comprises (a) a DNA prepared by ligating the tetracycline responsive sequence and the promoter sequence controlled by the tetracycline responsive sequence to the 5' upstream side of the synaptic transmission-controlling DNA, (b) a DNA prepared by ligating a DNA which inhibits transcription or translation of the DNA under certain condition, to the upstream of the tetracycline expression-regulating DNA ligated in such a way that it is placed under the control by the promoter which is activated nonspecifically or in certain nerve cell under a specific condition, and (c) a DNA prepared by ligating a factor inducing the specific condition as described above in such a way that it is placed under the control of the promoter which is activated in certain nerve cell. It is preferable to prepare these 3 DNAs as separately independent DNA fragments or a plasmid where these fragments are inserted. It is also possible to prepare 1 DNA fragment by aligning 3 or 2 fragments, or prepare a plasmid into which such DNA fragment is inserted.

Preferable arrangement of these DNAs is those as described above. However, as long as the expression of the synaptic transmission-controlling protein can be reversibly controlled by controlling the presence or absence of tetracycline or its amount in the cell, these DNAs may be moved to other site or its sequence may be changed. Moreover, it is preferable that these transcription units contain at least 1 intron.

As the tetracycline responsive sequence and the promoter sequence controlled by the tetracycline responsive sequence, those described in (i) above can be used. In addition, among the promoter which is activated nonspecifically or in certain nerve cell, and controls the expression of the tetracycline expression-regulating DNA, a preferably usable promoter which functions in any cell includes β-actin promoter.

On the other hand, the examples of usable mechanism for inhibiting or releasing the expression of the tetracycline expression-regulating factor that is induced by this promoter, under certain condition, include Cre-lox system (Sternberg, N., and Hamilton, D., (1981) J. Mol. Biol., 150, 467-486 ; Sauer, B., and Henderson, N., (1988) Proc. Natl. Acad. Sci. USA, 85, 5166- 5170; Gu, H., et al., (1994) Science, 265, 103-106) and FLP-FRT system (Golic, K. G., and Lindquist, S. L., (1989) Cell , 44, 499-509). In the case of using the Cre-lox system, the DNA is used which is prepared by ligating a stop codon that is ligated with lox sequence in its both ends, between the nonspecific promoter and the tetracycline expression-regulating DNA as described above. Moreover, as the factor which induces certain condition, a Cre recombinase is used which has the activity of deleting a region which is placed between loxP sequences, by DNA recombination. The DNA encoding the factor is ligated to the downstream of the promoter which is activated in certain nerve cell described in (i) above.

### (iii) Construction of the expression control unit of the synaptic transmission-controlling protein in certain nerve cell using a stress protein promoter

A further example of the gene expression control unit comprises (a) a DNA prepared by ligating a promoter sequence of a proper stress protein, a synaptic transmission-controlling DNA ligated functionally to the promoter sequence, and a DNA which inhibits transcription or translation of the gene in the 5' side upstream of the DNA under certain condition, and (b) a DNA prepared by ligating a factor which induces the certain condition as described above in such a way that it is placed under the control of the promoter which is activated in certain nerve cell. It is preferable to construct these 2 DNAs as separately independent DNA fragments or as the plasmids into which each of these fragments is inserted respectively. However, it is possible to prepare 1 DNA fragment by aligning 2 fragments or a plasmid into which this fragment is inserted.

Preferable arrangement of these DNAs is that as described above. However, as long as the expression of the synaptic transmission-controlling protein can be reversibly controlled in the cell where these DNAs are transduced by controlling the presence or absence of suitable stress shock or the degree thereof , these DNAs may be moved to other site or its sequence may be changed. Moreover, it is preferable that these expression-controlling units contain at least 1 intron.

The stress protein promoter used in the expression-controlling unit of the present invention may be any one which can be activated by applying a proper stress to the cell where the unit has been introduced. Specifically, for example, a heat shock protein promoter is exemplified.

The examples of the mechanism for inhibiting or releasing the expression of the synaptic transmission-controlling DNA that is induced by this promoter, under certain condition, include Cre-lox system (Sternberg, N., and Hamilton, D., (1981) J. Mol. Biol. , 150, 467-486 ; Sauer, B. , and Henderson, N. , (1988) Proc. Natl. Acad. Sci. USA, 85, 5166- 5170; Gu, H., et al., (1994) Science, 265, 103-106) and FLP-FRT system (Golic, K. G., and Lindquist, S. L., (1989) Cell , 44, 499-509). In the case of using the Cre-lox system, a DNA can be used which is prepared by ligating a stop codon that is ligated with lox sequence in both ends thereof, between the stress protein promoter and the synaptic transmission-controlling DNA as described above. Moreover, as the factor which induces certain condition, Cre recombinase can be used which has the activity of deleting a region which is placed between loxP sequences by DNA recombination. The DNA encoding the factor is ligated to the downstream of the promoter which is activated in certain nerve cell described in (i) above.

In addition, there can be used a method for control such as a method of introducing a DNA having the construct wherein the synaptic transmission-controlling DNA is ligated to the downstream of the aforementioned stress protein promoter into the host, and applying the stress directly to certain nerve cell (Halfon, M. S., et al., (1997) Proc. Natl. Acad. Sci. USA, 94, 6255-6260; Halloran, M. C., et al., (2000) Development, 127, 1953-1960).

### (3) Introduction of the expression-controlling unit of the synaptic transmission-controlling protein into the host

The gene expression-controlling unit according to the present invention can be prepared by the method known per se, and can be introduced into a proper host to obtain a recombinant. The host into which the gene expression-controlling unit according to the present invention can be introduced, may be any one, so long as the unit can be introduced and the expression of the neurotransmitter release-controlling protein can be reversibly controlled in the recombinant. Specific examples thereof include a cultured cell such as PC12 (Greene, L., et al. , (1976) Proc. Natl. Acad. Sci. USA, 73, 2424-2428) , a nervous system cultured cell, a primary cell, a nervous system tissue slice culture, a fertilized egg, an embryonic stem cell, a nerve stem cell, and a living individual other than human.

The embryonic stem cell includes any cells capable of developing to an individual. The nerve stem cell includes any cells capable of differentiating to the nerve cell. The a living individual includes a nematode, Drosophila, zebra fish, avis such as foul, and mammals such as monkey, rat or mouse. Among them, a rodent is preferable and the mouse is more preferably used. As a line of the mouse, C57BL/6 line is preferably used in consideration of phenotype analysis of the created animal into which the aforementioned expression-controlling unit is transduced. In consideration of other factors including easiness of propagation, easiness of obtaining the fertilized egg and the line of an ES cell, other line can also be used.

As such the method for introducing the expression-controlling unit of the synaptic transmission-controlling protein according to the present invention into the host, the method known per se and ordinarily used can be used. In the case where the host is a cell such as a cultured cell, a nervous system cultured cell, a primary cell, a nervous system tissue slice culture, a fertilized egg, an embryonic stem cell, or a nerve stem cell, specific examples of the method include microinj ection method, calcium phosphate method, lipofection method, electroporation method, and virus infection method. On the other hand, in the case where the gene expression-controlling unit is composed of a plurality of DNA fragments, it is preferable to introduce these fragments each independently into the host by the method as described above. Introduction of a mixture of the plurality of DNA fragments is also possible. In particular, when the host is an animal which can be crossed, the expression-controlling unit of a whole can be constructed in the host body by the crossing of animals into which the DNA fragments have been independently introduced.

In the case where the host is an individual, a method which is ordinarily used for preparing a gene transformant in a host can be used. Examples thereof include the method in which the DNA is introduced into the above-mentioned fertilized egg and embryonic stem cell followed by development, the homologous recombination method, and the transposon method.

For the hosts subj ected to the DNA transduction procedure as described above, a method which is ordinarily used and is known per se, can be used for confirming achievement of actual introduction of a DNA of interest. Specific examples thereof include the method in which a drug-resistant gene is introduced together with the expression-controlling unit asdescribed above, the host is treated with the drug, and then a strain having resistance is selected; and the method in which the expression of a marker gene described in (2) above is used as an index. On the other hand, the confirmation can also be carried out by Southern blotting, RNA blotting or immunoblotting using DNA, RNA or a protein extraction solution prepared from a portion or all of the introduction host.

The thus obtained host into which each DNA fragment of the expression-controlling unit according to the present invention has been introduced, is occasionally simply referred to as "DNA transductant". By taking the case where the host is an animal individual such as mouse as an example, the method (method for preparing a transgenic animal) for introducing the expression-controlling unit of the synaptic transmission-controlling protein according to the present invention will be described below in detail.

### (4) Method for preparing a transgenic animal

For the production of the animal (which may be hereinafter referred to as "transgenic animal") into which the expression-controlling unit of the synaptic transmission-controlling protein is introduced among the DNA transductant according to the present invention, the gene expression-controlling unit mentioned in detail in (2) above, which requires no site-specific introduction, is treated to remove the unnecessary region such as a vector sequence portion used in cloning and is then purified. The gene expression-controlling unit is then injected into a pronucleus of the fertilized egg of an objective animal by the method as describedin (3) above, specificallybythemicroinjectionmethod. It is preferable to transplant the fertilized egg which has received injection to an oviduct of a false pregnant animal.

In the transgenic animal, a success of introduction of the expression-controlling unit as described above is evaluated by using an offspring originated from the transplanted fertilized egg. The evaluation method includes, for example, the method in which a tail of the offspring after weaning is cut out, a genome DNA is purified and fragmented with a proper restriction enzyme, and then Southern hybridization is carried out by using a probe which can recognize the nucleotide sequence of the introduced expression-controlling unit according to the present invention. Alternatively, the nucleotide sequence of the introduced expression-controlling unit according to the present invention can be confirmed by amplification by PCR.

One example of the thus-created animals is a mouse into which each DNA fragment of the gene expression-controlling unit according to the present invention has been introduced. Specific examples thereof include a mouse (which may be hereinafter referred to as "Tet mouse") into which the tetracycline expression-regulating DNA is introduced in such a way that it is controlled by the nerve cell specific promoter; and a mouse (which may be hereinafter referred to as "TransReg mouse") into which the DNA which encodes the synaptic transmission-controlling protein in such a way that its expression can be controlled, is introduced.

On the other hand, in the case where it is desired to site-specifically introduce the gene into the animal, the introduction can be carried out by using a targeting vector in which the DNA having a homology with the DNA sequence of the target position of the host for introduction was added in front of and behind the DNA fragment of the expression-controlling unit according to the present invention. Specifically, the introduction can be carried out by inducing homologous recombination in the embryonic stem cell of the animal by using the method described in Thomas, K. R., et al. , (1987) Cell, 51, 503-512 and then obtaining a chimera animal by the method which is conventionally used.

### (5) Construction of the gene expression-controlling unit in the transgenic animal individual

By crossing the transgenic animal (F0) obtained as described above with the wild type animal of the same line, F1 can be obtained. Whether or not the introduced DNA is transferred into the obtained F1, can be examined by using themethoddescribed in (4) above. Subsequently, propagation and passage are carried out to produce F2 and an F3, and the presence or absence of the introduced DNA is confirmed by analyzing the genome DNA of the offspring obtained in the same way. If a line of the animal having the DNA which has certain nucleotide sequence can be produced in this passage, the propagation and passage can be carried out in an ordinary keeping environment. By crossing the thus-obtained transgenic animals into which each DNA fragment of the gene expression-controlling unit according to the present invention are introduced, the gene expression-controlling unit according to the present invention can be constructed in a recombinant host.

In the linedtransgenic animals as described above , whether or not the introduced gene is actually transcribed and translated to a protein in the individual body and whether or not transcription and translation are carried out in the target region, can be analyzed and confirmed by the ordinarily used method such as RNA blotting by using an RNA extracted from each tissue or the like, in situ hybridization, Immunoblotting by using the protein extracted from each tissue, or immunochemistry.

### (6) Method for controlling the expression of the synaptic transmission-controlling protein in the DNA transductant

The DNA transductant according to the present invention can reversibly control the expression of the synaptic transmission-controlling protein in the transductant by placing the transductant under the condition which is suitable for the gene expression-controlling system which has been introduced. The condition which is suitable for the gene expression-controlling system means, for example, in the case where the used gene expression-controlling system employs a tetracycline gene expression-controlling system, the regulation of administration of a substance which regulates the transcription activating ability of the tetracycline expression-regulating factor such as doxycycline.

Specifically, in the case where the host is a cell, the expression of the protein can be reversibly controlled by adjusting addition, no addition or the concentration of the substance to be added to a culture solution of the cell. On the other hand, in the case where the host is a living organism individual, the expression of the protein can be reversibly controlled by administration or no administration of the substance to the DNA transductant. For the method of administration of the substance to the individual, it is preferred to mix the substance with a feed or water and administer the mixture. However, direct injection into a digestive tract, intraabdominal injection or direct administration into a brain can also be employed. In the case of carrying out the control of the synaptic transmission-controlling protein in the cell, the tissue or the slice originated from the aforementioned living organism individual into which the DNA has been introduced, it is preferred to carry out the control by regulating the concentration of the above described substance in the culture medium.

In the case where the gene expression-controlling system which is used is a heavy metal ion expression-controlling system, the condition described in Mayo, K. E., et al., (1982) Cell, 29, 99-108; Brinster, R. L., et al., (1982) Nature, 296, 39-42; Searle, P. F., et al., (1985) Mol. Cell. Biol., 5, 1480-1489 or the like can be used. In the case of the heat shock gene expression-controlling system, the condition described in Nouer, et al., (1991) Heat Shock Response, Nouer, L., Florida, Boca Raton, CRC, 167-220; Halfon, M. S., et al., (1997) Proc. Natl. Acad. Sci. USA, 94, 6255-6260; Halloran, M. C. , et al., (2000) Development, 127, 1953-1960 or the like can be used. Inaddition, in the case of the system is a hormone gene expression-controlling system, the reversible control of the gene expression can be carried out by the condition described in Lee, F. , et al., (1981) Nature, 294, 228-232; Hynes, N. E., et al., (1981) Proc. Natl. Acad. Sci. USA. , 78, 2038-2042; Klock, G. , et al. (1987) Nature, 329, 734-736; Israel, D. I., & Kaufman, R. J., (1989) Nucleic Acid Res., 17, 4589-4604 or the like.

### (7) Method of analysis of a phenotype which appears in the DNA transductant by controlling the expression of the synaptic transmission-controlling protein

The phenotype which appears in the DNA transductant by the reversible control of the expression of the synaptic transmission-controlling protein, can be analyzed by analyzing a difference in a biochemical, physiological, morphological, behavioral change or the like, which appears in a cell, a tissue, a living organism individual or the like, between before and after the regulation or between the DNA transductant and the wild type, when the expression level of the synaptic transmission-controlling protein was controlled in said DNA transductant by the method described in (6) above. The phenotype can be analyzed by properly combining known methods which are ordinarily used.

The phenotype to be analyzed in the present invention includes all the phenotypes of which difference is caused by the presence, absence or degree of the expression of the synaptic transmission-controlling protein, such as those which appear in the cell and the living organism individual and can be visually observed and those which requires physical and chemical analysis. Among these phenotypes, those which can be visually observed include a shape of the transductant which is judged by a morphological analysis, and when the host is an animal, its behavior, motor ability, learning ability, habit, and the like. On the other hand, phenotypes judged by physical and chemical analysis include an intracellular electric potential, an electric potential of extracellular membrane, a capacitance of the membranes, an amount of the released neurotransmitter, a chemical change and an expression amount of biological functional molecules such as a nucleic acid, protein, lipid or saccharide.

Examples of themethodof theanalysis include observations with a light microscope, electron microscope or by unaided eyes, when the phenotype to be analyzed is the shape of the transductant as described above. When the animal behavior is analyzed, maze test or rota-rod, or conditioned preference test, which are ordinarily used as the means for analysis of the animal behavior, are exemplified. In addition, in the case where the intracellular electric potential, the electric potential of extracellular membrane, the capacitance of the membranes, or the like are observed as the phenotype, an electrophysiological analysis method which are ordinarily used, is used. In the case where the phenotype is a chemical change or a quantitative change of biological functionalmolecules such as nucleic acid, protein, lipid, or saccharide, the analysis can be carried out by RNA blotting, RT-PCR, in situ hybridization, DNA chip, Immunoblotting, two-dimensional electrophoresis, chromatography, immunochemistry or mass spectrometry.

### (8) Method of analysis by using the host into which the expression-controlling unit of the nerve cells transmission function-controlling protein is introduced

If the DNA transductant according to the present invention is used, a function of certain nerve cell can be analyzed. For example, by controlling the expression of the synaptic transmission-controlling protein, if the above described phenotype, appearing when signal transmission between other cells receiving a signal from certain nerve cell is reversibly changed, is analyzed, a cause-effect relationship between the function and the phenotype of certain nerve cell can be revealed.

If the DNA transductant according to the present invention is used, the function of certain biological functional molecule can be analyzed. The "biological functional molecule" means a substance which influences the function of a living organism, such as nucleic acid, protein, lipid, or saccharide. The method of analysis of these functions includes, for example, the method of analyzing the physical changes such as the quantitative change, structural change or intracellular localization change, and the chemical changes such as a degree of chemical modification, e. g. , phosphorylation, or distributional change of the biological functional molecule in the nerve cell and other cells. The aforementioned changes occurs when the expression control of the synaptic transmission-controlling protein is carried out in certain nerve cell by the method according to the present invention and the signal transmission between other cells receiving the signal from the nerve cell is reversibly changed. By carrying out these analytical method, the cause-effect relationship between the phenotype appearing in the living organism and the function of the biological functional molecule can be revealed.

Moreover, when another different certain gene, an antisense polynucleotide of certain gene, antibody, a drug, or the like is introduced or administered to the DNA transductant according to the present invention from outside, a substance having certain physiological activity can be screened by analyzing its effect on the phenotype of the transductant.

The DNA transductant according to the present invention can be various disease model cells or disease model animals on the basis of phenotype and genes thereof. Specific examples of the diseases showing pathological state of these models include, for example, mental diseases such as schizophrenia and depression, and nerve degeneration diseases such as Parkinson's disease and Alzheimer's disease. By performing the phenotype analysis as described above for this disease model, the function analysis of the nucleic acid and other biological functional molecule and the screening of a substance having a physiological activity, an agent for prevention or therapy of the diseases showing the pathological state of this disease model can be screened.

The transgenic animal which carries the expression-controlling unit of the synaptic transmission-controlling protein according to the present invention can be crossed with another transgenic animal or a host which shows characteristic phenotype. By analyzing the phenotype of an offspring produced by crossing or the qualitative and quantitative changes of the biological functional molecule such as the nucleic acid, the disease model of the disease in which a plurality of genes are involved can be produced, and a substance having certain physiological activity can be screened by using this model.

### Examples

The present invention is described below in detail by the Examples. These Examples are presented for the purpose of concretely explaining the present invention, and do not limit the technical scope of the present invention. The genetic engineering technology used in the examples are those described in Molecular Cloning Laboratory manual 2nd edition Cold Spring Harbor Laboratory Press, Sambrook, J. , Fritsch, E. F. , Maniatis, T., unless otherwise stated.

### Example 1: Construction of the DNA encoding a Tetracycline-dependent transcription-regulating factor

### (1) Cloning of mouse GABA_{A}a6 gene expression-regulating region (1-1) Preparation of Exon1 probe

For the cloning of mouse GABA_{A}a6 gene expression-regulating region, a forward primer (MYK 141: SEQ ID No. 1, prepared by requesting Hokkaido System Science Co.) for a sequence from 1240th "g" to 1260th "A" of the same gene Exon1 registered in Gen Bank Accession No.: AJ222970, and a reverse primer (MYK 142: SEQ ID No. 2, prepared by requesting Hokkaido System Science Co.) for a sequence from 1628th "C" to 1648th "g" of the same, were prepared, and PCR was carried out by using the genome of C57BL/6 mouse as a template. The PCR reaction solution which was attached to LAPCR Kit Ver.2.1 (TAKARA Co. made) was used, and PCR was carried out in accordance with the manual attached to the kit. The genome DNA was prepared from a tail of a wild C57BL/6 mouse in accordance with the method of Example 3(3) (3-1) mentioned below.

This PCR yielded a specifically amplified band of approximately 400 bp. This band was cut out, purified and subcloned into pCR 2.1 vector (Invitrogen Co. made). This plasmid was named pCRExon1 vector.

Subsequently, the sequence of an insert region of pCRExon1 was analyzed by using a fluorescent multi-capillary sequencer CEQ2000 (Beckman Instruments made) , and it was confirmed that this insert contains the nucleotide sequence from 1240th to 1648th of Gen Bank Accession No. :AJ222970 with a lack of 1590th "C" of AJ222970.

### (1-2) Screening by Exon1 probe

pCRExon1 prepared in (1-1) above was cleaved with restriction enzyme EcoRI (TOYOBO o. made) , and a DNA fragment containing the insert was purified. The purified DNA fragment of approximately 400 bpwas used as a template to prepare aP32-dCTP (Amersham Pharmacia Biotech made)-labeled hybridization probe (Exon1 probe) by the multi-prime method using BcaBEST Labeling Kit (TAKARA made).

By using this Exon1 probe, 92160 clone contained in genome BAC library derived from a 129SVJ mouse ES cell: Mouse BAC filter Release II (Genome Systems Co. made) was screened by hybridization, and as a result 9 positive clones (Grid 7, field 2, 21-f, 308; Grid 7, field 3, 6-j, 333; Grid 7, field 3, 16-1, 315; Grid 7, field 5, 1-g, 329; Grid 8, field 1, 15-O, 337; Grid 9, field 4, 23-f, 400; Grid 9, field 4, 24-b, 388; Grid 9, field 5, 9-b, 425; Grid 10, field 1, 6-h, 451) were obtained

### (1-3) Obtaining of GABA_{A}a6 expression-regulating region

From the aforementioned 9 positive clones, Grid 7, field 5, 1-g, 329 was selected, and an approximately 7. 3 kb DNA fragment from the 5' side SphI restriction enzyme site to the 3' side EcoRI in Exon 8 (corresponding to the sequence from nucleotide number 1 to 7305 of Fig.1 ②; GenBank Accession No.: AJ222970) which contains GABA_{A}a6 expression-regulating region, was subcloned into pUC18 (TOYOBO made.) This plasmid was named pGABAPr.

As a result of the analysis of the total nucleotide sequence of the subcloned region by using a fluorescent multi-capillary sequencer: CEQ2000 (Beckman Instruments) , it was confirmed that the DNA of interest was obtained. In this analysis, there was a sequence site which is different from the nucleotide sequence of AJ222970 gene. This difference is considered to be caused by species difference between the mouse used for the analysis of the nucleotide sequence of AJ222970 and the mouse of the genome BAC library used for the present experiment.

### (2) Obtaining of the rtTA gene having the IRES sequence (Internal Ribosome Entry Site) added to its 5'side

The rtTA gene having the Encephalomyocarditis virus-derived IRES (Internal Ribosome Entry Site: 277 to 874 of Gen Bank Accession No.: X74312) added to its 5' side, was obtained by repeating PCR for subsequently adding a partial region of the IRES sequence to the 5'upstream region of ATg translation initiating sequence of the rtTA gene as the forward primer sequence. The primers which were used, were all those prepared by requesting Hokkaido System Science Co.

The primers which were used were the forward primer (MYK151a to 151i: SEQ ID No. 3 to 11, prepared by requesting Hokkaido System Science Co.) corresponding to the IRES sequence from 277th "C" to 874th "g" of Gen Bank Accession No.: X74312 and the reverse primer (MYK152: SEQ ID No. 12, prepared by requesting Hokkaido System Science Co.) corresponding to the Sacl region or its vicinity in the rtTA gene. pTet-On vector (Clontech made) was used as the template for PCR using MYK151a (SEQ ID No. 3) and MYK152. For the second and following PCR using MYK151b/ MYK152, the plasmid prepared by subcloning the DNA fragment amplified by previous PCR was purified and used as the template. The forward primer (MYK151a) was designed in such a way that the ATg sequence (12th ATg in the IRES region) of 872-874 of the IRES nucleotide sequence registered in Gen Bank Accession No. : X74312 is consistent with the ATg translation initiating sequence of the rtTA gene , and also that the nucleotide sequence "ggC" is inserted beneath this ATg translation initiating sequence and NcoI (CCATgg) restriction enzyme site is introduced without changing the reading frame of the rtTA gene(glycine is inserted as an amino acid sequence).

MYK151i (SEQ ID No. 11, prepared by requesting Hokkaido System Science Co.) has a nucleotide sequence to which a linker sequence is added to the 5' upstream side of the IRES sequence as described above. By PCR using MYK151i/ MYK152 as the primer, a DNA fragment of approximately 1470 bp was amplified. The DNA fragment was subcloned into pCR2.1 vector (Invitrogen Co. made.), and the resultant vector was named pSEQ2i vector.

Next, by using MYK151 (SEQ ID No. 13, prepared by requesting Hokkaido System Science Co.) that is the forward primer for introducing 3 restriction enzyme sites of SphI, EcoRI, and AflII (Bst98I) and a Stop codon sequence for 3 reading frames to the 5' side upstream of the IRES sequence from the 5' side, and MYK152 as the primer, PCR was carried out by using the pSEQ2i vector obtained as described above as the template. A DNA fragment of approximately 1470 bpwas amplifiedbythis PCR. This fragment was subcloned into pCR 2.1 vector (Invitrogen Co. made), and the resultant vector was named pSEQ2.

### (3) Construction of DNA for the expression of the Tetracycline-dependent transcription-inducing factor which is controlled by the GABA_{A}a6 promoter

In rtTA gene encoded by pTet-On vector (Clontech Co. made), SphI restriction enzyme site (gCATg/C) exists (1615thnucleotide site in pTet-On). When the nucleotide "T" in this SphI restriction enzyme site is substitutedwith "T", SphI restriction enzyme site can be eliminated while the encoded amino acid sequence is kept. MYK149 (SEQ ID No. 14, preparedby requesting Hokkaido System Science Co.) is the forward primer for carrying out this nucleotide substitution.

MYK150 (SEQ ID No. 15, prepared by requesting Hokkaido System Science Co.) is the reverse primer for introducing the restriction enzyme site in the order of NotI, BglII and EcoRI from the 5' region in the 3' side downstream region of SV40 polyA signal sequence.

When the pTet-On vector (Clontech Co. made) was used as the template and PCR was carried out by using the above-described MYK149 (SEQ ID No. 14, prepared by requesting Hokkaido System Science Co.) and MYK150 (SEQ ID No. 15) as the primers, a DNA fragment of approximately 700bp which contains the 3' portion of rtTA and the SV40 polyA signal sequence was amplified. This DNA fragment was subcloned into the pCR 2.1 vector (Invitrogen Co. made) to prepare pSEQ1 vector.

The obtained pSEQ1 vector was cleaved with SacI and EcoRI and the approximately 700bp fragment was purified, and then subcloned into SacI/EcoRI region of pUC18 vector (TOYOBO Co. made) to prepare pVECl vector.

The obtained pSEQ2 vector was cleaved with SphI and SacI and the approximately 1460 bp fragment was purified and subcloned into SphI/SacI region of pVECl vector (Toyobo Co . made) toprepare pVEC2 vector. The pVEC2 vector is the vector containing a sequence wherein IRES, rtTA and SV40 late polyA signal sequence are functionally connected from the 5' side in the cloning site of pUC18.

Subsequently, by using the obtained pVEC2 as the template, PCR was carried out by using the forward primer: MYK154 (SEQ ID No. 16, prepared by requesting Hokkaido System Science Co.) corresponding to PmaCI restriction enzyme site or its vicinity in the IRES region and the reverse primer :MYK155 (SEQ ID No. 17, prepared by requesting Hokkaido System Science Co.) for adjusting the 11th ATg sequence in IRES region to the Atg translation initiating sequence of the rtTA. By this amplification, a DNA fragment of approximately 300bp which contains PmaCI restriction enzyme site or its vicinity in the IRES region to 11th ATg sequence (Atg translation initiating sequence) in the IRES region, was obtained. This DNA fragment was named Fragment A.

On the other hand, PCR was carried out by using the forward primer: MYK156 (SEQ ID No. 18, prepared by requesting Hokkaido System Science Co.) for adjusting the 11th ATg sequence in the IRES region to the ATg translation initiating sequence of rtTA and the reverse primer: MYK158 (SEQ ID No. 19, prepared by requesting Hokkaido System Science Co.) corresponding to BsiWI (SplI) restriction enzyme site or its vicinity in rtTA gene, and using the obtained pVEC2 as the template. As a result, a fragment of approximately 700bp was amplified which covers the region from the 11th ATg sequence or its vicinity (ATg translation initiating sequence) in the IRES region to the BsiWI(SplI) restriction enzyme site or its vicinity in rtTA gene. This DNA fragment was named Fragment B.

The aforementioned Fragment A and Fragment B contain a sequence overlapping in the 11th ATg sequence (ATg translation initiating sequence) or its vicinity in the IRES region. By utilizing this overlapping sequence, PCR ligation of Fragment A and Fragment B was carried out.

A mixture solution of Fragment A and Fragment B was used as the template, and PCR was carried out by using MYK154 (SEQ ID No. 16, prepared by requesting Hokkaido System Science Co.) and MYK15 8 (SEQ ID No. 19, prepared by requesting Hokkaido System Science Co.) as the primer. As a result, a fragment of about 900bp was amplified. After purification, this fragment was subcloned into pCR 2.1 vector (Invitrogen Co. made) to prepare pAB vector, and was subjected to DNA sequencing by using the fluorescent multi-capillary sequencer: CEQ2000 (Beckman Instruments. Thus, it was confirmed that Fragment A was correctly ligated to Fragment B.

The prepared pAB vector was cleaved with PmaCI and BsiWI (SplI), and then, a fragment of approximately 900 bp was purified and ligated into the pVEC2 vector which was cut out by PmaCI/BsiWI (SplI). The prepared vector was named pVEC2(11) vector.

pGABAPr vector obtained (1-3) above was cleaved with SphI and Bst98I (AflII) to obtain a fragment of approximately 7.2 kbp fragment. This fragment was purified and ligated to the SphI/AflII (Bst98I) region of the above described pVEC2 (11) to obtain pVEC6(11). pVEC6(11) is a vector which contains the GABA_{A}a6 expression-regulating region, IRES, rtTA, SV40 polyA signal sequences which are functionally connected from the 5' side in the cloning site of the pUC18. This construct was shown in Fig. 1 ①.

### Example 2: Construction of the transgene encoding the nerve cell transmission function-controlling protein

### (1) Obtaining of a neurotoxin gene

### (1-a) Obtaining of neurotoxin gene (BoNT) derived from Clostridium botulinum

Neurotoxin (BoNT) gene of Clostridiumbotulinum was kindly presented in the form of the following plasmids by Professor Syunji Kosaki of Osaka Prefecture University.

| | |
|---|---|
| pBN3 | BoNT.A |
| pBN13 | BoNT.B |
| pBN27 | BoNT.Cl |
| pBN17 | BoNT.E |

### (1-b) Cloning of neurotoxin (TeNT) gene derived from Clostridium tetani

Neurotoxin (TeNT) gene derived from Clostridium tetani was obtained by culturing (collaborated by Prof. Mitsuaki Nishifuchi, Southeast Asia Research Center, Kyouto University) and boiling Clostridium tetani KZ1174 strain (kindly presented from Prof. Shinichi Nakamura, Lab. of Microbiology, Kanazawa University School of Medicine) in the culture solution for 15 minutes, and then applying PCR method using MYK121 (SEQ ID No. 20, prepared by requesting Hokkaido System Science Co.) and MYK126 (SEQ ID No. 21, prepared by requesting Hokkaido System Science Co.) primers and using the above billed solution. A DNA fragment of approximately 1. 4 kbp was amplified by this PCR, and the fragment was purified and subcloned into pCR2.1 vector (Invitrogen Co. made) to prepare pCRTeNT vector.

MYK121 (SEQ ID No: 20) is the forward primer for adding MluI restriction enzyme site to the 5' upstream region of the ATg translation initiating codon of TeNT light chain gene. MYK126 (SEQ ID No. 21) is the reverse primer for adding Not I restriction enzyme site to the 3' downstream region of the TeNT light chain gene. For designing the sequences of MYK121 and MYK126 primers, the registered sequence of Gen Bank Accession No.: X04436 was referred.

### (2) Construction of DNA for the Tetracycline-dependent expression of the synaptic transmission-controlling protein

MYK098 (SEQ ID No. 22) is the forward primer for adding SacII restriction enzyme site and Kozak sequence (Kozak, M., (1986) Cell, 44, 283-292) to the 5' upstream region of ATg translation initiating codon of a EGFP gene. MYK099 (SEQ ID No. 23) is the reverse primer for adding MluI restriction enzyme site to the 3' downstream region of AAg sequence which encodes lysine of the amino acid sequence number 239 of dlEGFP gene in pdlEGFP-N1 vector (Clontech Co. made).

By performing PCR using the pdlEGFP-N1 as the template and MYK098 and MYK099 as the primers, a DNA fragment of approximately 750 bp was amplified. This fragment was cleaved with SacII and MluI, and the thus-obtained purified fragment of approximately 740 bp was ligated to SacII/ MluI restriction enzyme site of pTRE2 vector. The prepared vector was named pTREEGFP vector.

MYK119 (SEQ ID No. 24, prepared by requesting Hokkaido System Science Co.) is the forward primer for adding (i.e., regulating a reading frame to a protein) NotI restriction enzyme site and one nucleotide of "T" to the 5' upstream region of AgC sequence encoding serine of the amino acid number 242 of dlEGFP gene, in this order from the 5' side. MYK101 (SEQ ID No. 25, prepared by requesting Hokkaido System Science Co.) is the reverse primer for adding XbaI restriction enzyme site to the 3' downstream region of TAg translation terminating codon of the dlEGFP gene. By performing PCR using the pdlEGFP-N1 vector as the template and MYK119 and MYK101 as the primers, a DNA fragment of approximately 150bpwas amplified. This fragment was cleaved with NotI and XbaI, and the resultant purified fragment of approximately 140 bp fragment was ligated to NotI/XbaI restriction enzyme site of pTREEGFP. The prepared vector was named pTREEGFPd1 vector.

The following PCR was carried out to add MluI for cloning and NotI to 5' terminal and 3' terminal, respectively, of BoNT gene which was obtained in (1-a) above. The forward primers which were prepared by adding MluI restriction enzyme site to the 5' upstream region of the ATg translation codon of each BoNT light chain gene, were used. For BoNT.A, BoNT.B, BoNT.Cl, and BoNT.E, MYK102 (SEQ ID No. 26), MYK105 (SEQ ID No. 27), MYK103 (SEQ ID No. 28) and MYK091 (SEQ ID No. 29) were respectively prepared by requesting Hokkaido System Science Co. The reverse primers which were prepared by adding the NotI restriction enzyme site to the 3' downstream region of each BoNT light chain gene, were used. For BoNT.A, BoNT.B, BoNT.Cl, and BoNT.E, MYK107 (SEQ ID No. 30), MYK110 (SEQ ID No. 31), MYK108 (SEQ ID No. 32) and MYK096 (SEQ ID No. 33) were respectively prepared by requesting Hokkaido System Science Co. The plasmid obtained in (1-a) was used as the template DNA, was and PCR was carried out in combination of the primers against each BoNT as described above.

By this PCR, the following BoNT genes were amplified. A DNA fragment of approximately 1.4 kbp of BoNT.A, a DNA fragment of approximately 1.3 kbp of BoNT.B, a DNA fragment of approximately 1.4 kbp of BoNT.Cl, and a DNA fragment of approximately 1.3 kbp of BoNT.E, were amplified. These fragments were purified, and then subcloned into pCR2.1 vector (Invitrogen Co. made).

The thus obtained plasmids to which each BoNT gene was subcloned, were cleaved with MluI and NotI to obtain DNA fragments of approximately 1.3 to 1.4 kbp. These fragments were purified, and then ligated to the MluI/NotI restriction enzyme site of the pTREEGFPd1 vector obtained as described above. The thus prepared vectors were named pTREBoNT.Ad1, pTREBoNT.Bd1, pTREBoNT.Cld1, pTREBoNT.Ed1, and pTRETeNTd1, respectively. These vectors contain a region wherein PhCMV promoter containing the tetracycline responsive sequence; a gene encoding the neurotoxin protein to which EGFP and PEST sequence of mouse ornithine decarboxylase are fused to its N-terminal side and its C-terminal side respectively; and rabbit β-globin polyA signal sequence, are functionally aligned. This construct was shown in Fig. 2. An intron is contained around rabbit β-globin polyA signal sequence.

### Example 3: Production of transgenic mouse

### (1) Preparation of a DNA sample for injection into a pronucleus of mouse fertilized egg

pVEC6 (11) prepared in the above Example 1 (3) was cleaved with SphI and NotI, and the cleaving reaction solution was subjected to electrophoresis in 1% low melting point agarose gel (Life Technologies Co. made: (CatalogNo. 15517-014) /0.5×TBE (0.045 M Tris-borate, 0.001 M EDTA). The agarose gel corresponding to approximately 9.3 kbp was cut out and was melted at 70 °C, and then treated with GELase (EPICENTRE TECHNOLOGIES Co. made) at 45 °C for 40 minutes and subjected to phenol chloroform treatment and ethanol precipitation to purify a DNA fragment of approximately 9.3 kbp. The DNA after ethanol precipitation was dissolved in TE (10mM Tris (pH8.0), 1mM EDTA), and then absorbance was measured at 260 nm for estimation of the concentration, and the concentration was adjusted to 10ng/µl by using calcium-and magnesium-free Dulbecco's PBS (Phosphate Buffered Saline: Life Technologies Co. made). This sample was used for gene transduction to mouse fertilized eggs.

pTREBoNT.Ad1, pTREBoNT.Bd1, pTREBoNT.Ed1 and pTRETeNTd1 obtained in the above Example 2(2) were cleaved with XhoI and SapI, and the DNA fragment of approximately 4 kbp was prepared in the same way as described above. This sample was used for gene transduction to mouse fertilized eggs.

In addition, pTREBoNT.C1d1 obtained in the above Example (2-2) was cleaved with AatII and SapI. AatII restriction enzyme site was used in place of XhoI, since there is XhoI restriction enzyme site in BoNT.Cl gene. The obtained DNA fragment of approximately 4 kbp DNA fragment was prepared in the same way as described above. These samples were each used for gene transduction to mouse fertilized eggs.

### (2) Gene injection into the pronucleus of mouse fertilized eggs

The fertilized eggs at a pronucleus stage which were obtained by crossing of C57BL/6 mice each other, were collected by oviduct perfusion, and were used as sample eggs for use in injection operation. In Example 3(1) as described above, each DNA solution adjusted to 10ng/ µl was filled in a capillary for microinjection, and injected into the pronucleus of the fertilized eggs at pronucleus stage. The surviving eggs after injection were transferred to a fresh culture solution and washed, and then cultured in a carbon dioxide gas incubator up to a 2-cell stage embryo. The developed 2-cell stage embryos were transferred to the oviduct of female mouse as a foster parent inducing false pregnancy, and an offspring was obtained. The thus obtained offspring was subjected to sexuality confirmation and weaning at the 3rd week after the birth. The thus obtained transgenic mouse was subjected to individual identification at about 4th week after the birth, and the success or failure of the gene transduction was analyzed by Southern hybridization.

### (3) Test of the success or failure of the gene transduction : genome Southern blotting analysis

### (3-1) GABA_{A}a6-rtTA transgenic mouse

A tail end (about 1 cm) of the transgenic mouse into which GABA_{A}a6-rtTA was introduced and which was obtained in Example 3 (2) , was cut out and incubated in a dissolving buffer (KURABO Co. made) at 55 °C for 6 to 16 hours to dissolve almost all the part of the tail. A residue was removed from the dissolving solution by centrifugation, and the genome DNA was purified by phenol/chloroform treatment and ethanol precipitation. In order to decompose the contaminating RNA, the genome DNA was dissolved in TE containing 100ng/ ml RNase A (Nakalai Tesque Co. made).

The genome DNA purified from the tail as described above was cleaved with restriction enzymes BamHI and SphI, and subjected to electrophoresis in 0.9% agarose gel/ 1 x TAE (0.04 M Tris-acetate, 0.001 M EDTA), and then blotted onto a nylon membrane (GeneScreenPlus : NEN Lifescience Co. made) by capillary method using 0.4N NaOH, 0.6M NaCl as a blotting solution.

The blotted membrane was dried and subjected to prehybridization in a hybridization buffer solution (1 M NaCl, 50 mM Tris (pH7.5), 10% Dextran Sulfate, 200 µg/ ml sonicated salmon sperm DNA (STRATAGENE Co. made: Catalog #201190-81) , 1% SDS) at 60°C. Then, Exon1 probe of GABA_{A}a6 labeled with aP32-dCTP which was prepared in Example 1 (1) was added, and hybridization reaction was carried out at 60°C overnight. After hybridization, the membrane was washed twice with 2×SSC at 60°C, and then twice with 0.2×SSC, 0.1% SDS at 60 °C, with shaking. Then, autoradiography was carried out by BAS2000 or BAS5000 (Fuj i Photo Film Co. made). A representative result is shown in Fig. 3 ①.

In the wild type mouse, only the signal of approximately 14 kbp endogenous GABA_{A}a6 gene (arrow in Fig. 3①) was detected. In addition, the line in which a signal of approximately 8.1 kbp (arrow head of Fig. 3①) is detected is the transgene-positive mouse. 10 lines of the mouse to which GABA_{A} α 6-rtTA was introduced, were obtained in total. These mice were named GABA_{A} α6-rtTA mice.

### (3-2) treBoNT.Ad1, treBoNT.Bd1, treBoNT.C1d1, treBoNT.Ed1, and treTeNTd1 transgenic mouse

The tail end (about 1 cm) of the transgenic mouse into which treBoNT.Ad1, treBoNT.Bd1, treBoNT.C1d1, treBoNT.Ed1, or treTeNTd1 were introduced, and which was obtained in Example 3(2) , was cut out, and the genome was prepared in the same way as in the above (3-1). The genome DNA prepared from the transgenic mouse into which treBoNT.Ed1 was introduced, was cleaved with KpnI and XbaI, and the genome DNAs prepared from the transgenic mouse into which treBoNT.Ad1, treBoNT.Bd1, treBoNT.C1d1 or treTeNTd1 was introduced, were cleaved with KpnI and EcoRT, and then blotting was carried out.

As the probe used for Southern blotting as described above, a DNA fragment of EGFR gene was obtained by the following PCR. An oligo DNA for forward primer: MYK189 (SEQ ID No. 34,) which has the sequence in which EcoRI restriction enzyme site and Kozak sequence were added to the 5' upstream of the ATg translation initiating sequence of the EGFP gene from the 5' side, and an oligo DNA for reverse primer: MYK220 (SEQ ID No. 35) which has the sequence in which Hind III restriction enzyme site was added to the 3' downstream region of the EGFP gene, were respectively prepared by requesting Hokkaido System Science Co, and were used. By performing PCR using pdlEGFP-N1 vector as the template DNA, a DNA fragment of approximately 750 bp was amplified. This fragment was purified and subcloned into pCR2.1 vector (Invitorogen) to prepare the pCREGFP vector.

This pCREGFP vector was cleaved with EcoRI and HindIII, and a DNA fragment of approximately 720 bp was purified and subcloned into EcoRI and Hind III restriction enzyme site of pGEM-4Z vector (Promega Co. made) to prepare pGEMEGFP. The fragment of approximately 720 bp which was produced by cleaving this pGEMEGFP with EcoRI and Hind III was used as the template, and a hybridization probe (EGFP probe,) which was labeled with aP32-dCTP (Amersham Pharmacia Biotech Co. made) was prepared by multiprime method using BcaBEST Labeling Kit (TAKARA Co. made).

By using this EGFP probe, hybridization was carried out in the same way as in the above Example 3 (3-1). Of the results, a representative treTeNTd1 transgenic mouse is shown in Fig. 3②. No signal is detected in the wild type mouse. The lines in which the signal of approximately 2.3 kbp (test of treBoNT.Ed1 mouse) or approximately 3 kbp (test of treBoNT.Ad1, treBoNT.Bd1, treBoNT.C1d1, and treTeNTd1 mice) is detected, are the transgene-positive mice.

The mice to which about 4 Kbp XhoI/SapI fragments of pTREBoNT.Ad1, pTREBoNT.Bd1, pTREBoNT.Ed1 or pTRETeNTd1 were introduced, were named treBoNT.Ad1, treBoNT.Bd1, treBoNT.Ed1, and treTeNTd1 mice, respectively. The mouse to which about 4 Kbp AatII/SapI fragment of pTREBoNT.Cld1 was introduced, was named treBoNT.Cld1 mouse.

As the result, 5 lines of treBoNT.Ad1 mice, 8 lines of treBoNT.Bd1 mice, 6 lines of treBoNT.C1d1 mice, 1 line of treBoNT.Ed1 mice, and 3 lines of treTeNTd1 mice, were obtained in total.

### Example 4: Evaluation of the nerve cell transmission function-controlling protein in a cultured cell system

### (1) Action target of neurotoxin protease

In order to confirm that a protease activity of the neurotoxin which enables a synaptic transmission is maintained in the fusion protein encoded by DNA of EGFP gene, DNA of neurotoxin gene and DNA of PEST sequence gene which were contained in pTREBoNT.Ad1, pTREBoNT.Bd1, pTREBoNT.C1d1, pTREBoNT.Ed1 or pTRETeNTd1 vector that were prepared in the above Example 2 (2) , the vector which constantly expresses the neurotoxin protein contained in the aforementioned vector and the DNA which encodes a protein which is a substrate of the neurotoxin contained in the vector, were co-expressed in a cultured cell, and the cleavage of the substrate protein which was produced was analyzed.

As the substrate protein of Clostridium tetani or Clostridium botulinum, Syntaxin 1A (288 amino acid residues, calculated molecular weight 33.1 kD), SNAP25A: Synaptosomal Associated Protein of 25KD (206 amino acid residues, calculated molecularweight23.3kD) , and VAMP2: Vesicle Associated Membrane Protein (116 amino acid residues, calculated molecular weight 12.7kD) were used. These were called SNARE (soluble N-ethylmaleimide-sensitive factor attachment protein receptors) proteins, and are known to play an important role in the process of the fusion of a synaptic vesicle containing the neurotransmitter with a presynaptic membrane (Südhof, T.C., (1995) Nature, 375, 645-653).

The relationship the neurotoxin protease with its action target protein is shown in the following Table 1 and Fig. 4.

**Table 1**

| Toxin | Substrate protein | Cleavage Site | Calculated molecular weight (kD) of a fragment of N-terminal side after cleavage |
|---|---|---|---|
| BoNT.A | SNAP25A | 197Q/198R | 22.4 |
| BoNT.B | VAMP2 | 76Q/77F | 8.0 |
| BoNT.Cl | Syntaxin1A | 253K/254A | 29.3 |
| | SNAP25A | 198R/199A | 22.6 |
| BoNT.E | SNAP25A | 179R/180I | 20.5 |
| TeNT | VAMP2 | 76Q/77F | 8.0 |

The SNAP25A cleaving activity of BoNT.Cl requires an amount of the protein about 1000- or more fold than BoNT.A and BoNT.E. It is presumed that a main toxicity activity is based on Syntaxin 1A as the substrate.

### (2) Preparation of a fusion protein expression vector

The pTREBoNT.Ad1 vector prepared in the above Example 2(2) was cleaved with Sac II, and the resultant cleaved end of Sac II was smoothed with KOD DNA Polymerase (TOYOBO Co. made). Next, this vector was cleaved withMluI, and a fragment of approximately 730 bp was purified. This fragment is DNA of EGFP gene. This fragment was cleaved with Xho I, and then smoothed in the same way as described above, and inserted into the pCI vector (Promega Co. made) cleaved by MluI.

The aforementioned plasmid in which EGFP was inserted was cleaved with MluI/XbaI, and the DNA fragments of neurotoxin gene which were prepared by cleaving pCIBoNT.Ad1, pCIBoNT.Bd1 and pCIBoNT.Ed1 with MluI/XbaI were inserted.

Here, BoNT.Cl and TeNT have 2 XbaI sites in the DNA of gene. Therefore, insertion into the vector was carried out separately twice. pTREBoNT.C1d1 and pTRETeNTd1 vectors were cleaved with MluI/ XbaI, and the fragments of approximately 300bp and approximately 90bp were purified respectively. These fragments were inserted in MluI/ XbaI restriction enzyme site of the aforementioned expression vector in which EGFP was inserted. The obtained plasmid was further cleaved with XbaI, and a fragment of approximately 1200 bp was purified. This fragment was each inserted in the XbaI restriction enzyme site of the expression vector prepared in the above in which a part of the toxin gene was inserted. In this ligation, the XbaI fragment of approximately 1200 bp can be ligated in 2 directions. However, a clone having the correct direction was selected by analyzing the nucleotide sequence.

### (3) Obtaining of the DNA encoding the substrate protein of the toxin, and preparation of the expression vector

### (3-1) DNA of Syntaxin 1A gene

DNA of Syntaxin 1A gene was obtained from a murine brain RNA by RT-PCR. A total RNA was purified from a brain tissue of a C57BL/6 mouse on the basis of the principle of AGPC (Acid Guanidinium-Phenol-Chloroform) method (Chomczynski, P., and Sacchi, N., (1987) Anal. Biochem., 162, 156-159) using RNAzol B (TEL-TEST Co. made).

From the purified murine brain total RNA, cDNA was synthesized by using Oligo dT-Adaptor Primer and AMV reverse transcriptase XL attached to RNA LA PCR Kit (AMV) Ver.1.1 (TAKARA Co. made) (reverse transcription reaction).

MYK201 (SEQ ID No. 36) as the forward primer for adding the MluI restriction enzyme site and the Kozak sequence to the 5' upstream region of the ATg translation initiating sequence of the Syntaxin 1A gene from the 5' side and MYK202 (SEQ ID No. 37 as the reverse primer for adding the SalI restriction enzyme site to the 3' downstream region of the TAg translation terminating sequence of the Syntaxin 1A gene were prepared by requesting Hokkaido System Science Co. The primers were designed with reference to the registered sequence of Gen Bank Accession No.: D45208.

PCR was carried out by using the cDNA which was prepared as described above as the template and using MYK201 and MYK202 as the primers. The amplified fragment of approximately 900 bp was purified, and subcloned into pCR2.1 vector (Invitrogen Co.) to prepare pCRSYN vector.

The aforementioned pCRSYN vector was cleaved with MluI/SalI, and a fragment of approximately 890bp was purified and then ligated into the MluI/ SalI restriction enzyme site of the pCI vector (Promega Co. made) to prepare the expression vector: pCISYN in which. DNA of the Syntaxin 1A gene was inserted.

### (3-2) DNA of SNAP25A gene

DNA of SNAP25A gene was obtained from the murine brain RNA by RT-PCR in the way as described above. For the primers for PCR, MYK205 (SEQ ID No. 38) as the forward primer for adding the MluI restriction enzyme site and the Kozak sequence to the 5' upstream region of the ATg translation initiating sequence of the SNAP25A gene from the 5' side and MYK208 (SEQ ID No. 39 as the reverse primer for adding the SalI restriction enzyme site to the 3' downstream region of the TAA translation terminating sequence of the SNAP25A gene were prepared by requesting Hokkaido System Science Co. The primers were designed with reference to the registered sequence of Gen Bank Accession No.: M22012.

PCR was carried out by using the cDNA which was prepared in (3-1) as described above as the template and using MYK205 and MYK208 as the primers. The amplified fragment of approximately 640 bp was purified, and subcloned into pCR2.1 vector (Invitrogen Co.) to prepare pCRSNAP25A vector.

The pCRSNAP25A vector was cleaved with MluI/ SalI, and the fragment of approximately 640 bp was purified, and ligated into the MluI/ SalI restriction enzyme site of the pCI vector (Promega Co. made) to prepare the expression vector: pCISNAP25A in which DNA of the SNAP25A gene was inserted.

### (3-3) DNA of VAMP2 gene

DNA of VAMP2 gene was obtained from the murine brain RNA by RT-PCR in the same way as in (3-1) above. For the primers for PCR, MYK212 (SEQ ID No. 40) as the forward primer for adding the MluI restriction enzyme site and the Kozak sequence to the 5' upstream region of the ATg translation initiating sequence of the VAMP2 gene from the 5' side and MYK213 (SEQ ID No. 41 as the reverse primer for adding the SalI restriction enzyme site to the 3' downstream region of the TAA translation terminating sequence of the VAMP2 gene were prepared by requesting Hokkaido System Science Co. The primers were designed with reference to the registered sequence of Gen Bank Accession No.: U60150.

PCR was carried out by using the cDNA which was prepared in (3-1) above as the template and using MYK212 and MYK213 as the primers. The amplified fragment of approximately 380 bp was purified, and subcloned into pCR2.1 vector (Invitrogen Co.) to prepare pCRVAMP2 vector.

The above pCRVAMP2 vector was cleaved with MluI/ SalI, and a fragment of approximately 380 bp was purified and ligated to the MluI/ SalI restriction enzyme site of the pCI vector (Promega Co. made) to prepare the expression vector: pCIVAMP2 in which DNA of the VAMP2 gene was inserted.

### (4) Transduction of DNA of gene into a cultured cell

The cultured strain cell, a COS7 cell (ATCC:CRL1651) derived from a kidney of an African green monkey was cultured in DMEM (Dulbecco's Modified Eagle Medium) culture medium (Life Technologies Co. made) containing a 10% bovine fetus serum (Clontech Co. made) in10% CO₂ at 37 °C.

For gene transduction to the cultured cell, SuperFect reagent (QIAGEN Co. made) was used. For gene transduction, the COS7 cell cultured as described above was inoculated in 6-well culture plate at the cell density of 5 X 10⁵ cells/ well, and was cultured for 24 hours under the same condition.

The toxin protein-fused protein expression vector prepared in the Examples 4 (2) and (3) was mixed with the substrate protein expression vector of the toxin in a proper mixing ratio. The total amount of the DNA to be used was 2.4µg/well, and the volume was adjusted to 100µl by adding serum-free DMEM. To this diluted DNA mixture solution was added 14.4µl SuperFect reagent, and the resultant was mixed well, and incubated at a room temperature for 5 to 10 minutes. Moreover, 600µl of DMEM containing 10% serum was added to prepare a gene transductuion solution. To the COS7 cell which was washed once with serum-free culture medium, was added 714.4µl of the gene transduction solution in total per well, and the cells were culture at 37 °C. After 3 hours, the culture medium was removed, and 2 ml of DMEM containing 10% serum was added, and culturing was continued for 48 hours.

The expression vectors used for coexpression of a toxin protein and an action target protein of the toxin were the following 6 combinations.
Toxin protein expression vector / action target protein expression vector
pCIBoNT.Ad1/pCISNAP25A
pCIBoNT.Ed1/pCISNAP25A
pCIBoNT.Bd1/pCIVAMP2
pCITeNTd1/pCIVAMP2
pCIBONT.C1d1/pCISYN
pCIBoNT.C1d1/pCISNAP25A

### (5) Analysis of the protein which is expressed in a gene DNA-transduced cell

After the above described cells were cultured, a culture plate was placed on ice and the medium was removed. Then, PBS (Phosphate Buffered Saline: TAKARAmade) at 4 °C was added, and the cells were collected by using a cell scraper. A cell suspension was transferred to a 1.5 ml tube, and the cells were washed by repeating three times each of precipitation of the cells by using 10 k rpm centrifugation and suspension in the PBS at 4 °C. After the final centrifugation, the precipitated cells were suspended in 1 × Tris-Glycine based sample buffer solution (100m M Tris (pH6.8), 2% SDS, 10% glycerol, 0.002% bromophenolblue, 1%β-mercaptoethanol) or 1 × Tris-Tricinebased sample buffer solution (133 mM Tris (pH6.8), 1.3% SDS, 26.7% glycerol, 0.03% Coomassie Brilliant Blue-G250, 1% β-mercaptoethanol), and boiled at 100 °C for 3 minutes to solubilize the protein. Thus, the sample for SDS-PAGE (Sodium Dodecyl Sulfate-Poly-Acrylamide Gel Electrophoresis) was obtained.

The electrophoresis was carried out at 20 to 50 mA for 2 to 6 hours by using 12.5% polyacrylamide gel and Tris-Glycine buffer solution (25 mM Tris, 192 mM Glycine, and 0.1% SDS) for the SDS-PAGE separation of the protein extraction solution of the cell to which DNAs of the Syntaxin 1A and SNAP25A genes were transduced, which was obtained in (4) above, or by using 17.5% polyacrylamide gel and Tris-Tricine buffer solution (10mM Tris, 10 mM Tricine, and 0.01% SDS) for the SDS-PAGE separation of the protein extraction solution of the cell to which DNA of VAMP2 gene was transduced.

The gel after the aforementioned SDS-PAGE was equilibrated in the blotting buffer solution (25 mMTris (pH9. 5) , 192 mMGlycine, 20% methanol, 0.05% SDS) , and was blotted on a PVDF membrane (Immobilon-P:Millipore Co. made) at 100 V for 1 hour with cooling the blotting buffer solution at 4 °C.

A filter after blotting was soaked in 100% methanol, and then washed with PBS. Then, blocking was carried out in PBST (PBS containing 0.1% Tween-20) containing 1% skim milk (Yukijirushi Co. made) at a room temperature for 30 minutes in order to prevent non-specific adsorption of a protein. The filter was incubated with a primary antibody solution diluted with PBST containing 1% skim milk at the room temperature for 1 hour, washed with PBST three times, and incubated with a secondary antibody solution diluted with PBST containing 1% skim milk at the room temperature for 45 minutes. In addition, the filter was washed with PBST three times, subjected to chemoluminescence reaction by ECL-plus (Amersham Pharmacia Biotech made,) , and exposed to an X-ray membrane: Hyperfilm-MP (Amersham Pharmacia Biotech made).

The antibody used and its dilution rate are mentioned below. In the detection of Syntaxin 1A, 1µg/ µl of Clone SP6 (Upstate Biotechnology Co. made: Catalog #05-397) was diluted 100,000 folds and was used as the primary antibody, and an HRP-conj ugated goat anti-mouse IgG antibody (Santa Cruz Biotechnology Co. made: Catalog SC-2005) was diluted 5000 folds, and was used as the secondary antibody. In the detection of SNAP25A, 4µg/µl of a goat anti-human (rat) SNAP25A polyclonal antibody (Santa Cruz Biotechnology Co. made: Catalog SC-7538 and SC-7539) was diluted 8000 folds and was used as the primary antibody, and an HRP conjugated camel anti-goat IgG antibody (Santa Cruz Biotechnology Co. made: Catalog SC-2020) was diluted 5000 folds and was used as the secondary antibody. In the detection of VAMP2, a rabbit anti-rat (mouse) VAMP2 polyclonal antibody (kindly presented by Dr. Masami Takahashi of Mistubishi Kagaku Institute of Life Sciences) was diluted 250 folds and was used as the primary antibody, and an HRP conjugated goat IgG antibody (Santa Cruz Biotechnology Co. made: Catalog SC-2020) was diluted 5000 folds and was used as the secondary antibody.

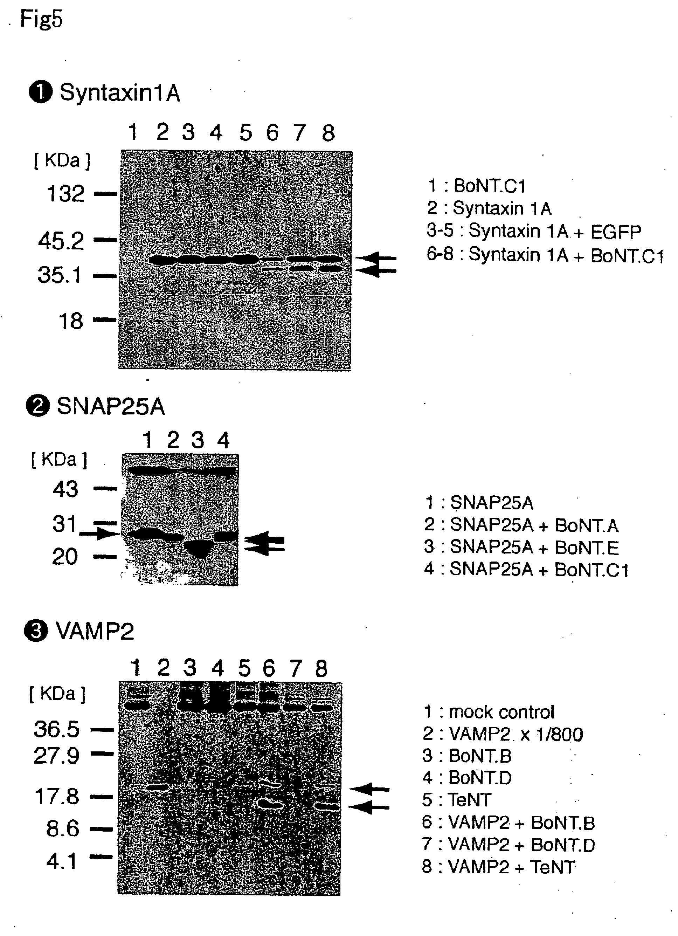

The result of the analysis of the protein expressed in these DNA-transduced cells is shown in Fig. 5.

It is presumed that all of the primary antibodies used above recognize the N-terminal side of the substitute protein which is produced by cleavage by a protease activity of the toxin. Fig. 4 shows the site where the substrate protein is cleaved when the toxin protein and the substrate protein are coexpressed in the cultured cell. From the result of Fig. 5, it was demonstrated that, even if the EGFP and PEST sequences were fused to the N-terminal and the C-terminal respectively, the toxin protein maintained the protease activity to cleave the substrate.

### Example 5: Expression analysis of the transgene in GABA_{A}a6-rtTA gene transduced mouse: mRNA northern blot analysis

GABA_{A}a6-rtTA gene transduced mouse is a transgenic mouse which was produced with a purpose to specifically express the rtTA gene in cerebellar granule cells. An expression analysis was carried out in order to confirm that rtTA gene was really expressed specifically in cerebellar granule cells of the GABA_{A}a6-rtTA gene transduced mouse which was produced.

### (1) Purification of mRNA

Using 8 lines (line numbers 612, 613, 615, 620, 621, 626, 628, and 606) among independent 10 lines, which were identified as the GABA_{A}a6-rtTA gene transduced mouse in Example 3, and a wild type mouse, the expression analysis of the rtTA gene was carried by RNA blotting analysis.

Brain tissues were obtained from the transgenic mouse of each adult line and the wild type mouse as a negative control, divided in 2 parts of a cerebellum and the other part of brain tissue , and total RNA was purified by using RNAzol™ B reagent (TEL-TEST) in accordance with the attached protocol. Tissues of a heart, liver, kidney, muscle and intestine were also obtained, and the total RNA was purified in the same way as in the brain tissue. Next, the mRNA was purified from the total RNA by using Oligotex™-dT30 <Super> reagent (JSR Co. and Roche Diagnostics Co. made) in accordance with the attached protocol. The concentration and purification degree of the purified mRNA was measured by measuring the absorbance at wavelengths of 260 nm and 230 nm, 280 nm, 320 nm.

### (2) Electrophoresis and blotting

Electrophoresis was carried out at 100 V for about 1 hour by using a solution which contains MOPS buffer solution (pH7.0) at a final concentration of 20 mM (1X) and 3.7 % formaldehyde, as the buffer solution for electrophoresis and loading the purified mRNA at 0.5 µg/well on a 1% agarose denatured gel (containing agarose at the final concentration of 1% in the buffer solution for electrophoresis). After electrophoresis, the gel was treated with 50 mM sodium hydroxide and 10 mM sodium chloride, neutralized with 0.1 M tris buffer solution (pH7.5), and equilibrated with 20 X SSC. Then, the gel was blotted on a positive charged nylon membrane (Roche Diagnostics Co. made) by capillary method using 20 X SSC as the blotting solution.

After blotting for 12 hours to 24 hours, the membrane was air-dried, and the mRNA which was transferred to the membrane was fixed by ultraviolet ray irradiation (120 mJ/cm²) (UV Stratalinker™: Stratagene).

The molecular weight was determined by using 0.24-9.5 kb RNA Ladder (Life Technologies Co. made) or DIG-labeled (0.3 - 6.9 kb) RNA marker (Roche Diagnostics Co. made).

### (3) Preparation of hybridization probe

The probe for the detection of the expression of rtTA gene was prepared as described below. When A of ATg of an initiation codon of the rtTA gene was assigned to the first residue, EcoRI restriction enzyme site (g/ AATTC) was added to the 5' side of the 648 bp gene fragment ranging from C of the 367th nucleotide to g of 1014th nucleotide by PCR, and Hind III restriction enzyme site (A/AgCTT) sequence was added to the 3' side thereof by PCR, and the resultant fragment was subcloned into EcoRI/Hind III region of pGEM-4Z vector (Promega Co. made) to prepare the pGEMrtTA (367-1014) vector. Next, the pGEMrtTA(369-1014) vector was linearized with EcoRI, and was purified to prepare a digoxigenin-labeled antisense RNA probe of rtTA gene by using T7RNApolymerase contained in DIG RNA Labeling Kit (Roche Diagnostics Co. made) in accordance with the attached protocol.

The probe for detection of the expression of β-actin gene was prepared as described below. An oligonucleotide DNA was synthesized, which has an antisense sequence against the 70 nucleotide region ranging from 438th A to 507th T of the nucleotide sequence registered in GenBank Accession No. : X03672. By using DIG Oligonucleotide Tailing Kit (Roche Diagnostics Co. made) in accordance with the attached protocol, a digoxigenin-labeled antisense oligonucleotide DNA probe of the β-actin gene was prepared.

The probe for the detection of the expression of a glyceraldehyde-3-phosphate dehydrogenase gene was prepared as described below. The oligonucleotide DNA was synthesized, which has the antisense sequence against the 70 nucleotide region ranging from 663rd C to 732nd T of the nucleotide sequence registered in GenBank Accession No. : M32599. By using the DIG Oligonucleotide Tailing Kit in accordance with the attached protocol, the digoxigenin-labeled antisense oligonucleotide DNA probe of the glyceraldehyde-3-phosphate dehydrogenase gene was prepared.

### (4) Hybridization, washing, detection, and stripping

The blotting membrane prepared in the Example 5(2) was prehybridized by using DIG EasyHyb Solution (Roche Diagnostics Co. made) , and then a digoxigenin-labeled probe against the gene to be detected for its expression was added, and hybridization was carried out. A hybridization temperature was 68 °C on use of the RNA probe and 42 °C on use of oligonucleotide DNA probe. After 12 to 24 hours, the blotting membrane was washed with 2 X SSC solution warmed to the hybridization temperature and next with 0.1 X SSC solution. Then, chemoluminescence reaction was carried out with CDP-Star reagent (Roche Diagnostics Co. made) by using DIG Block Buffer Set and DIG Luminescence Detection Kit (Roche Diagnostics Co. made) in accordance with the attached protocol, and the membrane was exposed to the X-ray membrane (Hypermembrane-ECL: Amersham Pharmacia Biotech made).

For performing hybridization using the blotting membrane once hybridized and another probe, the blotting membrane was washed twice in a solution of 50 % formamide, 1 % SDS and 50 mM tris [pH 8.0] at 72 °C for 30 minutes, rinsed with milli-Q water, and equilibrated with 2 X SSC solution. Then, a digoxigenin-labeled probe against the gene of which expression should be newly detected was added and re-hybridization was carried out.

### (5) Result of RNA blotting analysis in the GABA_{A}a6-rtTA gene-transduced mouse

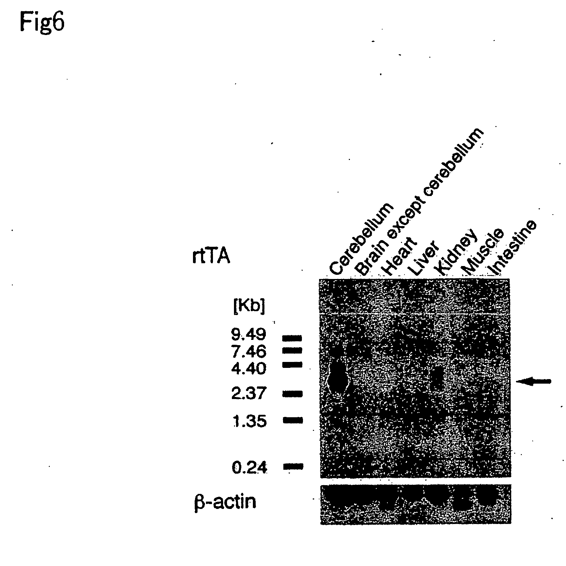

It is expected that an expression signal of the rtTA gene is detected around the position of approximately 3 kb which is a sum of the sequence sizes of an exons 1 to 8 of GABA_{A}a6 gene, IRES, rtTA gene and polyA. In all the 8 lines of the tested GABA_{A}a6-rtTA gene-transduced mouse, the expression signal of the rtTA gene was detected in cerebellum (arrow in Fig. 6) , and was not detected in any other brain tissue regions than the cerebellum. The expression signal of the rtTA gene was not detected at all in the cerebellum and other brain tissue regions than the cerebellum in the wild type mouse.

The strongest expression signal of the rtTA gene was found in the line of No.620, and this No.620 line was subjected to mRNA blotting analysis for the rtTA gene expression in other organs (heart, liver, kidney, muscle, and intestine) than the brain. In the line of No.620, the expression of mRNA of the rtTA transgene was found specifically in the cerebellum and was not detected at all in other brain regions than the cerebellum and other organs than the brain (Fig. 6).

Meanwhile, equalities of an amount and quality (whether or not the mRNA is decomposed) of the mRNA used for the mRNA blotting analysis for rtTA gene expression and an efficiency of operations such as blotting, were confirmed by performing the mRNA blotting analysis by using the expression amounts of the β -actin gene (Fig. 6, bottom) and the glyceraldehyde-3-phosphate dehydrogenase gene as internal controls.

The results described above shows that the rtTA gene was expressed specifically in cerebellum in the produced GABA_{A}a6-rtTA gene-transduced mouse.

### $Example 6: Expression analysis of a transgene in the GABA_{A}a6-rtTA gene-transduced mouse: In situ hybridization analysis

In Example 5, in the GABA_{A}a6-rtTA gene-transduced mouse, it was confirmed by the RNA blotting analysis that a transgene was expressed specifically in the cerebellum. Next, in order to examine the cells of the cerebellum where this rtTA gene was expressed, in situ hybridization analysis was carried out.

In situ hybridization analysis was carried out in 6 lines of the produced GABA_{A}a6-rtTA gene-transduced mouse (line Nos. 612, 613, 620, 621, 626, and 628) and the wild type mouse (the negative control).

### (1) Preparation of frozen sections of the brain tissue

The mouse was anesthetized with diethyl ether, and the brain was quickly removed and divided in a proper size. This brain tissue was embedded in Tissue-Tek^{R} OCT-Compound (Sakura Finetechnical Co. made) , and was rapidly frozen with isopentane cooled with dry ice. The frozen tissue block was stored at -20 to -80 °C. At sectioning, sections having a thickness of 10 µm were prepared by a cryostat of which inside temperature was adjusted to -20 to -14 °C, and were adhered to a MAS coated slide glass (Matsunami Co. made).

### (2) Prehybridization

The section adhered to the slide glass was fixed with a fixing solution (4 % paraformaldehyde and 0.1 M PBS), and was subj ected to acetylation in a solution of 0.25 % acetic anhydride, 0.9 % sodium chloride and 0.1 M triethanolamine [pH 8.0] in order to suppress the nonspecific reaction of the probe. The section was rinsed with PBS and was then subjected to defatting and dehydration in 70 %, 80 %, 90 %, 100 %, and 100 % ethanol series. The section was dried at 55 °C for 15 to 60 minutes, and was stored at -80 °C until hybridization.

### (3) Preparation of hybridization probe

As a template DNA used for preparation of a probe for in situ hybridization, pGEMrtTA (369-1014) which was linearized by the restriction enzyme EcoRI and was purified in Example 5 (2) was used. 0.5µg of the template DNA was reacted at 37 °C for 1 to 2 hours in a solution of 1 X Transcription buffer solution (40mMTris [pH8.0] , 50mMsodiumchloride, 8 mM magnesium chloride, 2 mM spermidine) , 500 µM ATP, 500 µM GTP, 500 µM TTP, 10 mM DTT, 11.5 U/ µl RNase inhibitor (Takara Co. made) , 20 µM S-CTP, 20 µM [a-³⁵S] CTP, andT7polymerase (Stratagene Co. made) , toprepare an antisense RNA probe of the rtTA gene labeled with [a-³⁵S]CTP. After the template DNA was decomposed with DNase (Promega Co. made) , unreacted mononucleotide and decomposed products of DNA were removed by Sephadex G-50 (Amersham Pharmacia Co. made) and ethanol precipitation. The solution was adjusted to be 0.5 to 1.0 X 10⁸ cpm/ ml with a hybridization buffer solution (50 % formamide, 2 X SSC, 100 mM tris [pH7.4], 10% dextran sodium sulfate, 0.2 % SDS, and 1 X Denhart Solution) to prepare the hybridization probe solution.

### (4) Hybridization, washing, and detection

To the section on the slide glass which was dried at 33 °C was added the hybridization probe solution of the antisense RNA probe of the rtTA gene, and hybridization reaction was carried out at 60 °C. After 12 to 24 hours, the section on the slide glass was washed with a solution of 2 X SSC and 10 mM β-mercaptoethanol at 60 °C, and the unreacted RNA probe was decomposed in a solution of 2 µg/ ml RNase, 10 mM tris [pH8.0] , 1 mM EDTA and 500 mM sodium chloride at 37 °C. Further, the section was washed with a solution of 0.2 X SSC and 10 mM β-mercaptoethanol at 60 °C, and was then subj ected to dehydration in the ethanol series to drying. The expression signal of the rtTA gene was detected by autoradiography using BAS5000 (Fuji Photo Film Co. made), Hyperfilm-ßMax (Amersham Pharmacia Co. made) and an emulsion (NTB3; Kodak Co. made).

### (5) Result of in situ hybridization analysis in the GABA_{A}a6-rtTA gene-transduced mouse

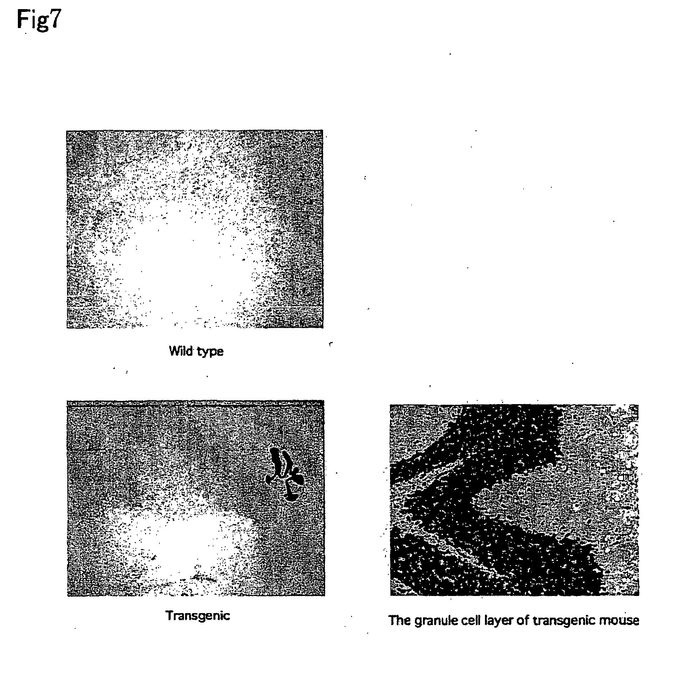

In all the tested 6 lines of the GABA_{A}a6-rtTA gene-transduced mouse, the expression signal of the rtTA was detected only in the cerebellar granule cells, and was not detected in a Pulkinje's cell and cells in a molecular layer (layer where a parallel fibers, axons of the granule cells, are present), a white matter, and a cerebellar nuclei region. Moreover, the expression signal of the rtTA was not detected in other brain regions including an inferior olive nucleus from which climbing fibers are originated (Fig. 7, Transgenic, The granule cell layer of transgenic mouse). As to the strength of the expression signal of the rtTA gene by in situ hybridization analysis, the strongest signal was detected in the line of No. 620, which corresponds to the result of the mRNA blotting analysis of Example 5 ("Transgenic" of Fig. 7 is the line of No.620). On the other hand, in the wild type mouse, the expression signal of the rtTA was not detected in all the regions including the cerebellar granule cells (Fig. 7, wild type).

From the above results, it was confirmed that the rtTA gene was expressed specifically in the cerebellar granule cells in the produced GABA_{A}a6-rtTA gene-transduced mouse.

### Example 7: Production of a double transgenic mouse having both the GABA_{A}a6-rtTA gene and the treToxin gene as the transgenes

The mice treBoNT.Ad1, treBoNT.Bd1, treBoNT.C1d1, treBoNT.Ed1, and treTeNTd1 obtained in Example 3 are referred to as treToxin mice.

It is expected that the offspring obtained by crossing the GABA_{A}a6-rtTA gene- transduced mouse with treToxin gene-transduced mouse includes a double transgenic mouse having both the genes as the transduced genes. Identification of the genotype of the offspring obtained by crossing was carried out by genome Southern blotting analysis as shown in Example (3), and as a result, the double transgenic mice were obtained almost in the proportion of the Mendel's law (i.e. , in the case where a gene-transduced animal having the heterozygous GABA_{A}a6-rtTA gene was crossed with a gene-transduced animal having the heterozygous treToxin gene, the probability in which the offspring were the double transgenic mice, is 25 %). This result shows that a defect of lethality (Eisel, U., et al. , (1993) EMBO J., vol.12, 3365-3372, Sweeney, S. T., et al., (1995) Neuron, vol.14, 341-351,) that appears when Clostridium tetani and Clostridium botulinum were expressed in a situation with constancy or a low temporal and spatial specificity, has been overcome in the double transgenic mice produced by us.

### Example 8: Expression induction and expression elimination of the neurotoxin protein in the GABA_{A}a6-rtTA gene- and treToxin gene- double transgenic mouse

The offspring of the GABA_{A}a6-rtTA gene-transduced mouse and treToxin gene-transduced mouse was weaned at about 4th week after the birth, and the genotype was determined by genome Southern blotting analysis.

For inducing an expression of the neurotoxin protein, doxycycline (Sigma Co. made) was added in drinking water (2 mg/mL doxycycline and 10% sucrose) and a pellet feed (Bio-Serve Co. made, 6 mg/ g doxycycline) , and was administered to the mice. When the induction of the expression of the neurotoxin protein is not desired, and when the expression induction should be stopped, both drinking water and the feed were replaced to those containing no doxycycline.

### Example 9: Detection of the expression induction and expression elimination of the neurotoxin protein in the GABA_{A}a6-rtTA gene-and treToxin gene- double transgenic mouse Immunoblotting method

### (1) Preparation of a brain tissue protein-solubilized solution

A brain tissue was resected from the mouse, and was divided in 2 parts of the cerebellum and the other brain tissue. The divided brain tissue was transferred to an Eppendorf tube, and was homogenized in PBS containing 4% SDS, 2 mM EDTA and a protease inhibitor cocktail tablet (Roche Diagnostics Co. made) (1 tablet/5 ml) by using a pestle of a plastic. After homogenization, the tissue was incubated in a water bath at 100 °C for 5 minutes, and centrifuged at 15000 rpm to precipitate an insoluble component and the genome DNA, and the supernatant was collected as a brain tissue protein-solubilized solution. A protein concentration of the brain tissue protein-solubilized solution was measured by using a DC Protein Assay reagent (BIO-RAD Co. made) constituted based on Lowry method in accordance with the attached protocol.

### (2) SDS-PAGE, blotting, antibody reaction, and detection

In the same way as in the case of Example 4(5), the brain tissue protein-solubilized solution was loaded in 40 µg/ protein lane, and SDS-PAGE was carried out and then blotting on a PVDF membrane was carried out. For the detection of the expression oftheneurotoxinprotein, apolyclonal antibody (Molecular Probe Co. made: Catalog A6455) against EGFP which was fused with an N-terminal side of the neurotoxin protein as a tag, was used as the primary antibody, and a HRP-conjugated goat anti-rabbit IgG antibody (Santa Cruz Biotechnology) was used as the secondary antibody, and the chemoluminescence reaction by ECL-Plus was carried out. It is expected from the calculated value of the amino acid composition that the expression signal of the neurotoxin protein is detected around 84 kDa.

### (3) Detection of expression induction and expression elimination of the neurotoxin protein in the GABA_{A}a6-rtTA gene- and treToxin gene- double transgenic mouse.

### Result of immunoblotting method

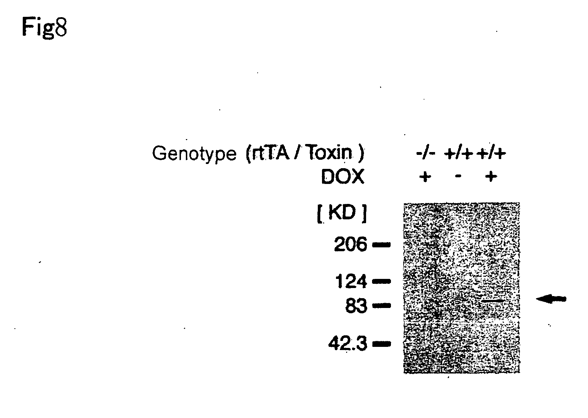

From the cerebellum of the wild type mouse (Genotype: -/-, DOX: +) to which doxycycline was administered for 1 week, the double transgenic mouse (Genotype: +/+, DOX: -) to which doxycycline was not administered, and the double transgenic mouse (Genotype: +/+, DOX: +) to which doxycycline was administered for 1 week, the protein-solubilized solution was prepared. Then, the expression induction of the neurotoxin protein was examined. The results are shown in Fig. 8. Only in the cerebellum of the double transgenic mouse (Genotype: +/+, DOX: +) to which doxycycline was administered for 1 week, the expression signal was detected around 84 kDa (Fig. 8, the arrow). These results showed that the expression of the neurotoxin protein were induced in the cerebellum by administering doxycycline for 1 week to the GABA_{A}a6-rtTAgene- and treToxin gene- double transgenic mouse by the method as described in Example 8, and also that the expression was not induced when doxycycline was administered to the wild type mouse or when no doxycycline was administered to the double transgenic mouse.

### Example 10: Detection of expression induction and expression elimination of the neurotoxin protein in the GABA_{A}a6-rtTA gene-and treToxin gene- double transgenic mouse

### Immunohistochemistry

It was found by the analysis of the expression induction of the neurotoxin protein by immunoblotting in Example 9 that the expression of the neurotoxin protein was induced in the cerebellum of the double transgenic mouse (Genotype: +/+, DOX: +) to which doxycycline was administered for 1 week. Then, the cell of the cerebellum, in which the neurotoxin protein is expressed and induced, was identified.

### (1) Preparation of a frozen section of the brain tissue

The mouse was anesthetized with diethyl ether, and was subj ected to thoracotomy. PBS was perfused from a left ventricle of the heart for 2 minutes, and a fixing solution (phosphate buffer solution [pH 7.3] containing 4% paraformaldehyde) was perfused for 10 to 15 minutes, each at a flow rate of 10 ml/min by using a peristaltic pump. The brain was resected, and then divided in appropriate sizes, soaked in the fixing solution to be subjected to penetration and fixation at 4 °C for 12 hours to 48 hours. The fixed brain tissue was transferred to PBS containing 30 % sucrose and 0.05 % sodium azide, and a cryoprotection operation was carried out twice at 4 °C for 12 hours to 48 hours by changing the solution for the purpose of suppressing the tissue damage which is caused by crystallization of ice produced when the tissue is frozen. The brain tissue subjected to cryoprotection was embedded in a Tissue-Tek^{R} OCT-Compound, and was frozen rapidly with isopentane cooled with dry ice. The frozen tissue block was stored at -20 to -80 °C. At sectioning, a section with a thickness of 40 µm was prepared by using the cryostat of which inside temperature was set to - 20 to -14 °C. The section was stored in PBS containing 0.05 % sodium azide at 4 °C.

### (2) Antibody reaction (ABC method and fluorescence double staining method)

The section was washed with a washing buffer solution (PBS containing 0.1 % Triton^{R}X-100), and was incubated in an antibody-diluted solution (PBS containing 0.1 % Triton^{R}X-100, 2 % normal goat serum (Vector Lab. Co. made) and 0.05 % sodium azide) at the room temperature for 1 hour to block non-specific antigen-antibody reaction.

The primary antibodies prepared by diluting an anti-EGFP rabbit polyclonal antibody (Molecular Probe Co. made: Catalog A6455), an anti-calbindinD-28K mouse monoclonal antibody (Sigma Co. made: Catalog C9848), and an anti-parvalbumin mouse monoclonal antibody (Sigma Co. made: Catalog P3088) with the antibody-diluted solution, were used.

In the ABC (Avidin Biotin Complex) method, a secondary antibody prepared by diluting a biotin-labeled goat anti-rabbit IgG (H+L) antibody with the antibody-diluted solution was used. In addition, after reaction with a solution prepared by diluting a complex of avidin and biotin-labeled horseradish peroxidase (VECTASTAIN^{R} ABC Kit : Vector Lab. made) with a washing buffer solution, a coloring reaction was carried out with a solution of 0.5 mg/ml 3, 3'- Diaminobenzidine, tetrahydrochloride (Doj in Kagaku Kenkyuusyo made), 0.006 % hydrogen peroxide and 50 mM tris [pH7.5]. The section was adhered to the slide glass, and then was embedded in MountQuick (Daidou Sangyou K.K. made).

In the fluorescence double staining, secondary antibodies prepared by diluting Alexa Fluor^{R} 488 goat anti-rabbit IgG (H+L) antibody and Alexa Fluor^{R} 594 goat anti-mouse IgG (H+L) antibody with the antibody-diluted solution, were used. The section was adhered to the slide glass, and was embedded in VectaShield (Vector Lab. made), and observation was carried out by using a laser scanning microscope system (LSM 510 META : Zeiss Co. made).

### (3) Detection of expression induction and expression elimination of the neurotoxin protein in the GABA_{A}a6-rtTA gene- and treToxin gene- double transgenic mouse: Results of immunohistochemistry method

Fig. 9 A, B, C and D shows the results of the analysis wherein the primary antibody reaction was carried out by using EGFP antibody and the ABC method was used in the secondary antibody reaction and thereafter, for the cerebellum section of the wild type mouse (6-week old) (Fig. 9A: Genotype: -/-, DOX: +) to which doxycycline was administered for 1 week, the double transgenic mouse (6-week old) (Fig. 9B: Genotype: +/+, DOX: -) to which doxycycline was not administered, the double transgenic mouse (6-weekold) (Fig. 9C: Genotype: +/+, DOX: +) to which doxycycline was administered for 1 week, and the double transgenic mouse (10-week old) (Fig. 9D: Genotype: +/+, DOX: + -> -) to which doxycycline was administered for 2 week followed by removing doxycycline for 3 weeks. In the double transgenic mouse (6-week old) (Fig. 9C: Genotype: +/+, DOX: +) to which doxycycline was administered for 1 week, a signal of the reaction to an anti-EGFP antibody which indicates the presence of the neurotoxin protein was detected in themolecular layer (layerwhere aparallel fibers which are axons of the granule cells are present) of the cerebellum and the granule cell layer (layer where cell bodies of the granule cells are present in high density). No reaction signal was detected in the Pulkinje's cell, and no staining area was found. Thereby, it was found that no neurotoxin protein is present. Under other 3 conditions (Fig. 9A, B, and D) , no reaction signal were detected at all.

In order to identify, in more detail, the expression distribution of the neurotoxin protein in the double transgenic mouse (6-week old) (Genotype: +/+, DOX: +) to which doxycycline was administered for 1 week, fluorescence double staining was carried out (Fig.9E, F, G, H, I and J). Photographs arranged in 3 rows were taken from the same observation field. The green fluorescent signals (Fig. 9 E and H) showing the presence of the neurotoxin protein were localized in the molecular layer (ML) and the granule cell layer (GL) and were not detected in the Pulkinje's cell layer. These observations coincide with the detection result by the previous ABC method. The red fluorescent signal (Fig. 9F) showing the presence of calbindinD-28K was detected in the Pulkinje's cell, and the red fluorescent signals (Fig. 9I) showing the presence of parvalbumin were detected in the Pulkinje's cell, the stellate cell, and a basket cell. By combining the green fluorescent signal (Fig. 9E and H) and the red fluorescent signal (Fig. 9F and I) which were originated from the same observation field (Fig. 9 G and I), it was confirmed that individual signals did not overlap with each other and were perfectly separated from each other. This fact means that the expression induction of the neurotoxin protein is localized in the granule cells of the cerebellum and is not expressed in the Pulkinje's cell, the stellate cell, and the basket cell.

In the result of in situ hybridization analysis of the expression of rtTA gene in Example 6, the expression signal of the rtTA gene was localized in the granule cells existing in the cerebellar granule cell layer, and was not detected in the white matter, the Pulkinj e' s cell, and the molecular layer. On the other hand, it was found from the result of example 10 that the expression signal of the neurotoxin protein was detected in the granule cell layer (layer where cell bodies of the granule cells exist in high density) and the molecular layer. The molecular layer is composed of the parallel fibers, which are axons of the granule cells existing in the overwhelming density, dendrites of the Pulkinje's cell, the stellate cell, the basket cell, and the like. Among these cells, no expression of the neurotoxin protein was found in dendrites of the Pulkinj e' s cell, the stellate cell, and the basket cell (see the result of fluorescence double staining (Fig.9E, F, G, H, I and J). Therefore, it is presumed that the expression signal of the neurotoxin protein detected in the molecular layer is derived from the parallel fibers that are an axons of the granule cells.

In conclusion, it was confirmed that, in the GABA_{A}a6-rtTA gene- and treToxin gene- double transgenic mouse, the rtTA gene was expressed specifically in the cerebellar granule cell, and the expression of the neurotoxin protein was induced specif ically in the cerebellar granule cell by administration of doxycycline, and the neurotoxin protein was transported from cell bodies of the cerebellar granule cells to the parallel fibers (an action site of the neurotoxin protein) of the axon and was distributed widely.

On the other hand, it was also confirmed that the induced expression of the neurotoxin protein was reversible, and even after the expression was once induced, the presence of the neurotoxin protein can be eliminated by eliminating doxycycline from drinking water and the pellet feed (Fig. 9D).

### Example 11: Analysis of behavioral change caused by administration of doxycycline to the GABA_{A}a6-rtTA gene- and treToxin gene- double transgenic mouse

When the neurotoxin protein is expressed in the cerebellar granule cell, the release of the neurotransmitter from the granule cell is suppressed to inhibit transmission of nerve signal from the granule cell to post-synapse such as the Pulkinje's cell. The influence of this phenomenon on the mouse individual was analyzed from the viewpoint of a behavior of the mouse, particularly an ability of a coordinated movement. An experiment of behavioral analysis was carried out with reference to Kadotani, H., et al., (1996) J. Neurosci. vol. 16, 7859-7867, .Carter, R. J., et al., (1999) J. Neurosci. vol.19, 3248-3257, Crawley, J. N., "What's wrong with my mouse ?", A John Wiley & Sons, Inc.

### (1) Analysis of a behavioral change by visual observation

The GABA_{A}a6-rtTA gene- and treToxin gene- double transgenic mouse showed the similar behavioral pattern to that of the wild type mouse in walking before administration of doxycycline. However, at about 1 week (period where the neurotoxin protein was expressed) after doxycycline was administered, the mouse showed phenotypes of coordinated movement disturbances, such as slow movement, staggering gait, and crawling gait, which is different from that of the wild type mouse. On the other hand, when doxycycline was administered to the wild type mouse, the mouse having only GABA_{A}a6-rtTA gene as a transgene, and the mouse having only treToxin gene as a transgene, the phenotypes as described above were not observed and these mouse could not be distinguished from the wild type mouse to which doxycycline was not administered. In addition, in the GABA_{A}a6-rtTA gene- and treToxin gene- double transgenic mouse, the phenotype of the above described coordinated movement disturbance was induced by administration of doxycycline for 1 to 2 weeks (the expression of the neurotoxin protein is induced specifically in the cerebellar granule cell) , and then the mouse was returned to the condition of no administration of doxycycline. After 3 weeks (the neurotoxin protein was no longer present in the cerebellar granule cell), an attitude was observed. As a result, the phenotype of the above described coordinated movement disturbance disappeared and the mouse could not be distinguished from the wild type mouse to which doxycycline was not administered.

Therefore, it is concluded that the phenotype of the coordinated movement disturbance is caused by simple existence of 2 genes, i. e. , GABA_{A}a6-rtTA gene and treToxin gene in mouse individual or, not by a side effect of doxycycline, but by expression and elimination of the neurotoxin protein which is induced in the cerebellar granule cell by administration and no administration of doxycycline in the GABA_{A}a6-rtTA gene- and treToxin gene- double transgenic mouse.

In order to further quantify this change, Rota-Rod test and Balance Beam test were carried out.

### (2) Behavior analysis by Rota-Rod test

The mouse was put on a rod (UGO BASILE Co. made) moving at a rotation speed of 15 r.p.m. (rotation/ min) or 30 r.p.m. The mouse shows the coordinated walking movement matching rotation of the rod in order to avoid falling down from the rod. The mouse that can not match (coordinated movement is disturbed) rotation of the rod falls down from the rod or is rotated together with the rod fastening on the rotating rod. The time (Staying time, unit is second) for coordinated walking movement of the mouse on the rod is regarded as a score to quantify the ability of the mouse. The test time for 1 trial was set to 60 seconds at most. Even if the mouse could stay over 60 seconds on the rotating rod, the test was stopped and the score was recorded as 60 seconds.

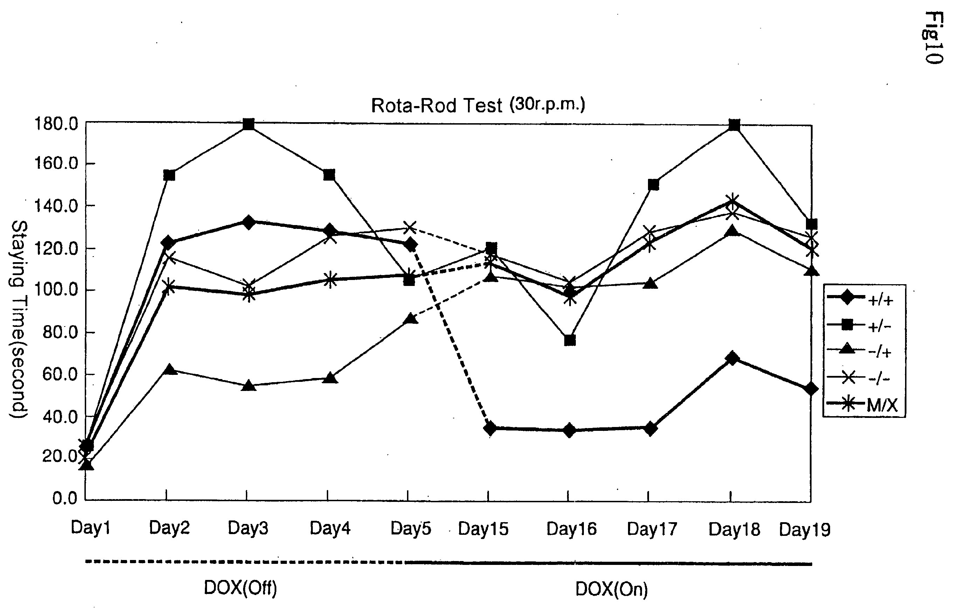

The wild type mouse (-/-) before doxycycline was administered, the transgenic mouse (+/-) having only GABA_{A}a6-rtTA gene, and the transgenic mouse (-/+) having only treToxin gene, and the GABA_{A}a6-rtTA gene- and treToxin gene-double transgenic mouse (+/+) , were put on the rotating rod (Fig. 10). At the first day of the test, the mouse is in an unfamiliar situation and hence could not achieve the coordinated walking movement on the rotating rod, and the mice rotate together with the rotating rod or fall down from the rod. As the result, the staying time on the rod became short in all the genotypes. However, the same test was repeated everyday and at the 5th day of the test, mice of any genotype became successfully operate the coordinated walking movement on the rotating rod and the staying time on the rod became long. However, when doxycycline was administered to the GABA_{A}a6-rtTA gene- and treToxin gene-double transgenic mouse, the mouse showed the phenotypes that the staying time on the rod became short and the coordinated walking movement was disturbed. On the other hand, when doxycycline was administered to the wild type mouse, the transgenic mouse (+/-) having only GABA_{A}a6-rtTA gene, and the transgenic mouse (-/+) having only treToxin gene, the following results were obtained. i.e. , the level of the staying time was the same as that before administration, and the coordinated walking movement was not disturbed.

The data series of MIX in Fig. 10 is those obtained by pooling the data of the wild type mouse (-/-,) the transgenic mouse (+/-) having only GABA_{A}a6-rtTA gene, and the transgenic mouse (-/+) having only treToxin gene.

### (3) Result of behavior analysis by a balance beam test

The mice were placed on a wooden rod with a width of 30 mm, 15 mm and 6 mm, which was placed transversely in a position with a height of 40 cm over a floor. The mice hated a high place and walked across the rod by walking in order to escape from the place into a safety cage (a paper-made box having floor width of 20 cm, floor depth of 16.5 cm and height of 10.5 cm; An entrance with a width of 8 cm and a height of 10.5 cm was provided in its front center. On the floor of the safety cage, wooden flooring chips ordinarily used for a keeping cage were filled) which was places at a distance of 60 cm from the place where the mouse was placed. If the mouse can keep a balance of the body and do coordinated walking movement, the mouse can successfully wake over the rod and escape into the safety cage. However, if the mouse fails to keep the balance of a body or fails to do the coordinated walking movement, the mouse shows the phenotypes such as falling down from the rod, failure to keep a correct attitude resulting in hanging, or fastening on the rod to fix its body, and can not escape into the safety cage. In the case where the mouse could successfully escape into the safety cage, evaluation was scored as "succeed". In the case where the mouse could not escape into the safety cage, evaluation was scored as "fail". Thus, the ability of passing the balance beam was scored. This test has a difficulty in such a degree that a normal mouse can pass for 10 seconds at most. Even if more time is required, the mouse can pass for 20 seconds at most. Therefore, the test time of 1 trial was set to be 60 seconds as most. In the case where the mouse can not escape into the safety cage within 60 seconds (in this situation, the mouse was fastened on the rod to fix its body in large numbers of cases) the test was stopped and the score was recorded as "fail".

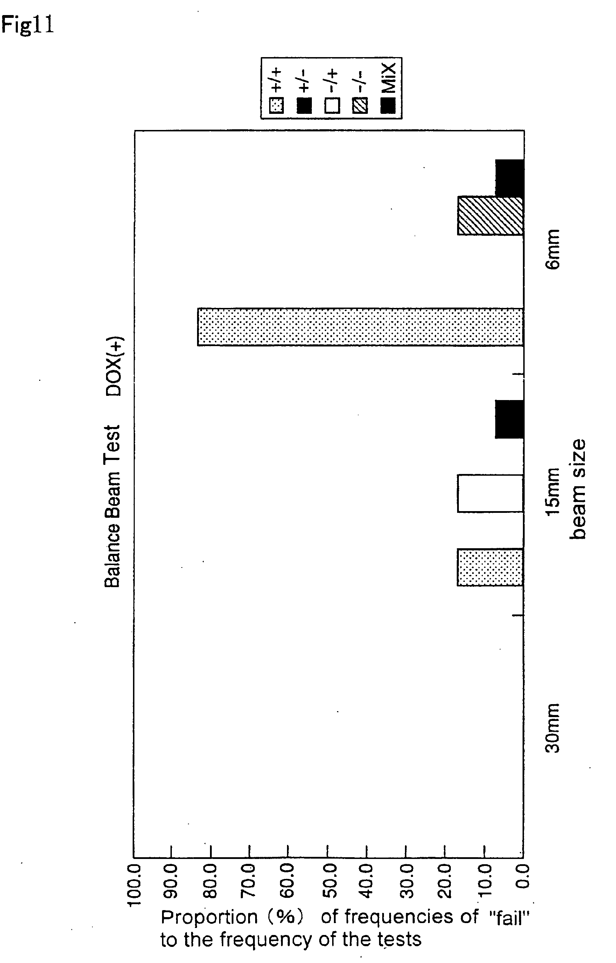

The graph of Fig. 11 shows a proportion of frequencies of "fail" to the frequency of the tests on an axis of ordinates, when the width of the rod was changed to 30 mm, 15 mm and 6 mm. When the width was set to be 30 mm, mice of any genotype passed easily on the rod and no "fail" was observed. When the width was changed gradually to 15 mm and 6 mm, mice of "fail" were observed. Particularly, in the 6 mm width, the GABA_{A}a6-rtTA gene- and treToxin gene- double transgenic mouse to which doxycycline was administered, showed the result of "fail" in 80% or higher tests. This fail ratio is a prominent increase as compared with the ratio of about 10% in the other genotypes to which doxycycline was administered.

The data series of MIX in Fig. 11 is those obtained by pooling the data of the wild type mouse (-/-,) the transgenic mouse (+/-) having only GABA_{A}a6-rtTA gene, and the transgenic mouse (-/+) having only treToxin gene.

### Industrial Applicability

By providing a host to which, a system which can reversibly control the expression of a protein having the synaptic transmission-controlling activity according to the present invention, has been introduced, it has become possible to perform a functional analysis of a nerve cell function, which was not so far accurately analyzed because its control is irreversible, or of a biological functional molecule such as genes expressed in said cell or a cell related to said cell.

## Claims

1. A DNA which comprises a DNA encoding a protein having a synaptic transmission-controlling activity and has a construct capable of controlling reversibly the expression of the protein having a synaptic transmission-controlling activity in certain nerve cell.

2. A gene expression control unit which comprises (a) a DNA construct which can reversibly control the expression of a protein having a synaptic transmission-controlling activity in certain nerve cell and (b) a DNA which is ligated in such a way that the DNA is placed under the control of said DNA construct and encodes the protein having a synaptic transmission-controlling activity.

3. A gene expression control unit which comprises (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity.

4. The gene expression control unit according to claim 3 wherein the protein having a neurotransmitter release - controlling activity is a neurotoxin.

5. A host having the DNA or the gene expression control unit according to any one of claims 1 to 4.

6. A method for analyzing the function of certain nerve cell, wherein the expression of the protein which is contained in the host according to claim 5 and has a neurotransmission function-controlling activity in certain nerve cell, is reversibly controlled, and the change of a phenotype appearing in the host is analyzed.

7. A method for analyzing the function of a biological functional molecule, wherein the expression of the protein which is contained in the host according to claim 5 and has a neurotransmission function-controlling activity in certain nerve cell is reversibly controlled, and the physical and chemical changes of the biological functional molecule in the nerve cell or a cell associated therewith are analyzed.

8. A method for analyzing the function of certain nerve cell, wherein (1) (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity, are introduced into a host, and (2) the change of a phenotype appearing in the host is analyzed by controlling the presence or absence of a specific stimulus against the host or the nerve cell or its strength.

9. A method for analyzing the function of a biological functional molecule, wherein (1) (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) a promoter which is controlled by the protein, and a DNA which is ligated in such a way that the DNA is placed under the control of the promoter and encodes a protein having a neurotransmitter release-controlling activity, are introduced into a host, and (2) the physical and chemical changes of the biological functional molecule in certain nerve cell or the cell associated therewith is analyzed by controlling the presence or absence of a specific stimulus against the host or the nerve cell or its strength.

10. A method according to claim 8 or 9, wherein the protein having a neurotransmitter release-controlling activity is a neurotoxin.

11. A fertilized egg, an embryonic stem cell and a nerve stem cell of an non-human animal which has (a) a transcription control region DNA which is activated specifically to certain nerve cell and a DNA which is ligated in such a way that the DNA is placed under said transcription control region, and encodes a protein which is activated by a specific stimulus and has an ability of activating a specific promoter and (b) apromoter which is controlled by the protein, and a DNA which is ligated in such a way that the DNA is placed under the control of the promoter and encodes a protein having a neurotransmitter release-controlling activity.

12. The fertilized egg, the embryonic stem cell and the nerve stem cell according to claim 11 wherein the non-human animal is a rodent.

13. The fertilized egg, the embryonic stem cell and the nerve stem cell according to claim 11 or 12 wherein the non-human animal is a mouse.

14. A transgenic non-human animal which is obtained by development from fertilized egg or the embryonic stem cell according to any one of claims 11 to 13, and an offspring thereof.

15. A transgenic animal cell which is obtained from the transgenic animal according to claim 14.

16. A nerve cell which is obtained by differentiation of the nerve stem cell according to any one of claims 11 to 13.


SEQUENCE LISTING
<110> Gencom
<120> Gene expression control unit and use thereof
<130> A21223A
<160> 41
<170> PatentIn Ver. 2.0
<210> 1
<211> 21
<212> DNA
<213> Artificial Sequence
<220>
<223> Description of Artificial Sequence:Synthesized
<400> 1
gcccattcga agtccatgcc a 21
<210> 2
<211> 21
<212> DNA
<213> Artificial Sequence
<220>
<223> Description of Artificial Sequences:Synthesized
<400> 2
catagaataa tgaagagcca g 21
<210> 3
<211> 112
<212> DNA
<213> Artificial Sequence
<220>

<223> Description of Artificial Sequence:Synthesized

<400> 3

aaaaacgtct aggccccccg aaccacgggg acgtggtttt cctttgaaaa acacgatgat 60

aatatggcca caaccatggc cacaaccatg ggctctagat tagataaaag ta 112

<210> 4

<211> 100

<212> DNA

<213> Artificial Sequence

<220>

<223> Description of Artificial Sequence:Synthesized

<400> 4

gtaccccatt gtatgggatc tgatctgggg cctcggtgca catgctttac atgtgtttag 60

tcgaggttaa aaaaacgtct aggccccccg aaccacgggg 100

<210> 5

<211> 100

<212> DNA

<213> Artificial Sequence

<220>

<223> Description of Artificial Sequence:Synthesized

<400> 5

gatagttgtg gaaagagtca aatggctctc ctcaagcgta ttcaacaagg ggctgaagga 60

tgcccagaag gtaccccatt gtatgggatc tgatctgggg 100

<210> 6

<211> 100

<212> DNA

<213> Artificial Sequence

<220>

<223> Description of Artificial Sequence:Synthesized

<400> 6

gcggccaaaa gccacgtgta taagatacac ctgcaaaggc ggcacaaccc cagtgccacg 60

ttgtgagttg gatagttgtg gaaagagtca aatggctctc 100

<210> 7

<211> 100

<212> DNA

<213> Artificial Sequence

<220>

<223> Description of Artificial Sequence:Synthesized

<400> 7

tgaagacaaa caacgtctgt agcgaccctt tgcaggcagc ggaacccccc acctggcgac 60

aggtgcctct gcggccaaaa gccacgtgta taagatacac 100

<210> 8

<211> 100

<212> DNA

<213> Artificial Sequence

<220>

<223> Description of Artificial Sequence:Synthesized

<400> 8

ttcccctctc gccaaaggaa tgcaaggtct gttgaatgtc gtgaaggaag cagttcctct 60

ggaagcttct tgaagacaaa caacgtctgt agcgaccctt 100

<210> 9

<211> 100

<212> DNA

<213> Artificial Sequence

<220>

<223> Description of Artificial Sequence:Synthesized

<400> 9

tgccgtcttt tggcaatgtg agggcccgga aacctggccc tgtcttcttg acgagcattc 60

ctaggggtct ttcccctctc gccaaaggaa tgcaaggtct 100

<210> 10

<211> 100

<212> DNA

<213> Artificial Sequence

<220>

<223> Description of Artificial Sequence:Synthesized

<400> 10

aacgttactg gccgaagccg cttggaataa ggccggtgtg cgtttgtcta tatgttattt 60

tccaccatat tgccgtcttt tggcaatgtg agggcccgga 100

<210> 11

<211> 90

<212> DNA

<213> Artificial Sequence

<220>

<223> Description of Artificial Sequence:Synthesized

<400> 11

actaactaac ggccgctacg taaattccgc cccccccccc cctctccctc ccccccccct 60

aacgttactg gccgaagccg cttggaataa 90

<210> 12

<211> 24

<212> DNA

<213> Artificial Sequence

<220>

<223> Description of Artificial Sequence:Synthesized

<400> 12

taagtggagc tcgtccccca ggct                                          24

<210> 13

<211> 53

<212> DNA

<213> Artificial Sequence

<220>

<223> Description of Artificial Sequence:Synthesized

<400> 13

gaggcatgcg aattccttaa gctaactaac taacggccgc tacgtaaatt ccg          53

<210> 14

<211> 47

<212> DNA

<213> Artificial Sequence

<220>

<223> Description of Artificial Sequence:Synthesized

<400> 14

gacgagctcc acttagacgg cgaggacgtg gcgatggcgc acgccga                 47

<210> 15

<211> 49

<212> DNA

<213> Artificial Sequence

<220>

<223> Description of Artificial Sequence:Synthesized

<400> 15

gaggaattca gatctgcggc cgcagcttgg tcgagctgat acttcccgt               49

<210> 16

<211> 24

<212> DNA

<213> Artificial Sequence

<220>

<223> Description of Artificial Sequence:Synthesized

<400> 16

gcggccaaaa gccacgtgta taag                                                24

<210> 17

<211> 48

<212> DNA

<213> Artificial Sequence

<220>

<223> Description of Artificial Sequence:Synthesized

<400> 17

acttttatct aatctagagc ccatattatc atcgtgtttt tcaaagga                       48

<210> 18

<211> 48

<212> DNA

<213> Artificial Sequence

<220>

<223> Description of Artificial Sequence:Synthesized

<400> 18

tcctttgaaa aacacgatga taatatgggc tctagattag ataaaagt                      48

<210> 19

<211> 21

<212> DNA

<213> Artificial Sequence

<220>

<223> Description of Artificial Sequence:Synthesized

<400> 19

aattgttttt cgtacgcgcg c                                                21

<210> 20

<211> 38

<212> DNA

<213> Artificial.Sequence

<220>

<223> Description of Artificial Sequence:Synthesized

<400> 20

tacaagacgc gtatgccaat aaccataaat aattttag                              38

<210> 21

<211> 42

<212> DNA

<213> Artificial Sequence

<220>

<223> Description of Artificial Sequence:Synthesized

<400> 21

ccatggctgc ggccgctgca gttctattat ataaattttc tc                         42

<210> 22

<211> 42

<212> DNA

<213> Artificial Sequence

<220>

<223> Description of Artificial Sequence:Synthesized

<400> 22

cagcctccgc ggcgccacca tggtgagcaa gggcgaggag ct 42

<210> 23

<211> 33

<212> DNA

<213> Artificial Sequence

<220>

<223> Description of Artificial Sequence:Synthesized

<400> 23

ggggggacgc gtcttgtaca gctcgtccat gcc 33

<210> 24

<211> 36

<212> DNA

<213> Artificial Sequence

<220>

<223> Description of Artificial Sequence:Synthesized

<400> 24

gagagagagc ggccgctagc catggcttcc cgccgg 36

<210> 25

<211> 36

<212> DNA

<213> Artificial Sequence

<220>

<223> Description of Artificial Sequence:Synthesized

<400> 25

ctcagctcta gactacacat tgatcctagc agaagc 36

<210> 26

<211> 46

<212> DNA

<213> Artificial Sequence

<220>

<223> Description of Artificial Sequence:Synthesized

<400> 26

tacaagacgc gtatgccatt tgttaataaa caatttaatt ataaag 46

<210> 27

<211> 46

<212> DNA

<213> Artificial Sequence

<220>

<223> Description of Artificial Sequence:Synthesized

<400> 27

tacaagacgc gtatgccatgt tacaataaat aattttaatt ataatg 46

<210> 28

<211> 46

<212> DNA

<213> Artificial Sequence

<220>

<223> Description of Artificial Sequence:Synthesized

<400> 28

tacaagacgc gtatgccaat aacaattaac aactttaatt attcag 46

<210> 29

<211> 43

<212> DNA

<213> Artificial Sequence

<220>

<223> Description of Artificial Sequence:Synthesized

<400> 29

tacaagacgc gtatgccaaa aattaatagt tttaattata atg 43

<210> 30

<211> 40

<212> DNA

<213> Artificial Sequence

<220>

<223> Description of Artificial Sequence:Synthesized

<400> 30

ccatggctgc ggccgcctta ttgtatcctt tatctaatga 40

<210> 31

<211> 41

<212> DNA

<213> Artificial Sequence

<220>

<223> Description of Artificial Sequence:Synthesized

<400> 31

ccatggctgc ggccgcttta acacttttac acatttgtat c 41

<210> 32

<211> 43

<212> DNA

<213> Artificial Sequence

<220>

<223> Description of Artificial Sequence:Synthesized

<400> 32

ccatggctgc ggccgcttta ttatataatg atctaccatc tat 43
<210> 33
<211> 48
<212> DNA
<213> Artificial Sequence
<220>
<223> Description of Artificial Sequence:Synthesized
<400> 33
ccatggctgc ggccgccctt atgcctttta cagaaacaat atttttac 48
<210> 34
<211> 38
<212> DNA
<213> Artificial Sequence
<220>
<223> Description of Artificial Sequence:Synthesized
<400> 34
gacgaattcg ccaccatggt gagcaagggc gaggagct 38
<210> 35
<211> 30
<212> DNA
<213> Artificial Sequence
<220>
<223> Description of Artificial Sequence:Synthesized
<400> 35
gagaagcttc ttgtacagct cgtccatgcc 30
<210> 36
<211> 40

<212> DNA

<213> Artificial Sequence

<220>

<223> Description of Artificial Sequence:Synthesized

<400> 36

cagctgacgc gtcgccacca tgaaggaccg aacccaggag                                40

<210> 37

<211> 33

<212> DNA

<213> Artificial Sequence

<220>

<223> Description of Artificial Sequence:Synthesized

<400> 37

tctagagtcg acctatccaa agatgccccc gat                                       33

<210> 38

<211> 40

<212> DNA

<213> Artificial Sequence

<220>

<223> Description of Artificial Sequence:Synthesized

<400> 38

tgacgcgtcg ccaccatggc cgaggacgca gacatgcgta                                40

<210> 39

<211> 34

<212> DNA

<213> Artificial Sequence

<220>

<223> Description of Artificial Sequence:Synthesized

<400> 39

gagtcgactt aaccacttcc cagcatcttt gttg                                34

<210> 40

<211> 37

<212> DNA

<213> Artificial Sequence

<220>

<223> Description of Artificial Sequence:Synthesized

<400> 40

gacgcgtcgc caccatgtcg gctaccgctg ccaccgt                             37

<210> 41

<211> 29

<212> DNA

<213> Artificial Sequence

<220>

<223> Description of Artificial Sequence:Synthesized

<400> 41

ggtcgactta agtgctgaag taaacgatg                                      29